(19) Europäisches Patentamt
European Patent Office
Office européen des brevets

(11) Veröffentlichungsnummer: **0 101 849**
**B1**

(12) **EUROPÄISCHE PATENTSCHRIFT**

(45) Veröffentlichungstag der Patentschrift: **03.06.87**

(51) Int. Cl.⁴: **A 61 K 31/715**, C 08 B 37/00

(21) Anmeldenummer: **83106734.3**

(22) Anmeldetag: **08.07.83**

(54) **Arzneimittel, Calciummischsalze von polymeren, anionischen Carbonsäuren und/oder ihren Schwefelsäureestern, Verfahren zu ihrer Herstellung und ihre Verwendung.**

(30) Priorität: **28.07.82 DE 3228231**

(43) Veröffentlichungstag der Anmeldung: **07.03.84 Patentblatt 84/10**

(45) Bekanntmachung des Hinweises auf die Patenterteilung: **03.06.87 Patentblatt 87/23**

(84) Benannte Vertragsstaaten: **AT BE CH DE FR GB IT LI LU NL SE**

(56) Entgegenhaltungen:
**DE - A - 2 759 032**
**FR - M - 3 068**
**FR - M - 6 649**
**GB - A - 13 777**
**GB - A - 1 125 924**
**GB - A - 1 394 741**

(73) Patentinhaber: **Algina Aktiengesellschaft, c/o Rensch Treuhand Baarer Strasse 43, CH-6301 Zug (CH)**

(72) Erfinder: **Kulbe, Klaus Dieter, Dr., Dipl.-Chem., Ritterstrasse 12, D-7031 Gärtringen 2 (DE)**
Erfinder: **Weber, Hans, Dr., Dipl.-Chem., Bittenfelderstrasse 8, D-7140 Ludwigsburg (DE)**

(74) Vertreter: **Weisert, Annekäte, Dipl.-Ing. Dr.-Ing. et al, Patentanwälte Kraus Weisert & Partner Thomas-Wimmer-Ring 15, D-8000 München 22 (DE)**

**EP 0 101 849 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein oral verabreichbares Arzneimittel zur Regulierung des Phosphat- und/oder Calciumspiegels bei chronischer Urämie und/oder zur Regulierung des Oxalat- und/oder Phosphatspiegels bei der Nierensteinprophylaxe, die Verwendung eines Calciumsalzes oder Calciummischsalzes einer natürlichen oder chemisch modifizierten polymeren anionischen Carbonsäure und/oder eines ihrer Schwefelsäureester, zur Herstellung eines oral verabreichbaren Arzneimittels sowie ein Calciummischsalz.

In der Bundesrepublik Deutschland gibt es zur Zeit etwa 14 000 Patienten mit chronischer Urämie, wobei die Tendenz steigend ist. Diese Patienten müssen sich dreimal wöchentlich für je drei bis sechs Stunden einer Blutwäsche nach einem der bekannten Verfahren, wie beispielsweise Hämodialyse oder Hämofiltration bzw. einer kontinuierlichen Peritonealdialyse, unterziehen, um urämische Toxine, wie zum Beispiel Harnstoff, Harnsäure, Creatinin etc., sowie Phosphat aus dem Organismus, d.h. aus dem Blut, zu entfernen. Trotz diätetischer Massnahmen ist jedoch der Phosphatspiegel bei den meisten Patienten auch nach dieser Blutreinigung noch zu hoch. Die Hyperphosphatämie ist ein wesentlicher Faktor in der Pathogenese der sekundären Hyperparathyreoidismus bei der Entwicklung der renalen Osteopatie unter chronischer Dialysebehandlung. Ursachen dafür sind die Bindung von Phosphat an bestimmte Proteine und die intestinale Resorption von Nahrungsphosphat und Phosphat aus der Gallenflüssigkeit. Deshalb sind fast alle Urämiker auf eine zusätzliche Medikation zur Senkung des Phosphatspiegels in den Normbereich angewiesen.

In den letzten Jahren wurden überwiegend Aluminiumhydroxide, zum Beispiel Aludrox ®, Antiphosphat ®, Alucap ® und Phosphonorm ®, zur intestinalen Phosphatbindung bei diesen Patienten verwendet. Dabei wurden bis zu 10 g und mehr pro Tag verabreicht (M.B. Kaye, Arch. Intern. Med. 124, 656 (1969)). In der DE-OS 2 618 083 wird ein weiteres aluminiumhaltiges Salz zur Reduktion der Phosphatresorption beschrieben.

Diesen bekannten Mitteln zur Phosphatbindung haften jedoch Nachteile an. Durch partielle Lösung des Aluminiumhydroxids ($Al(OH)_3$) im Magenmilieu zu $Al(OH)Cl_2$ und $AlCl_3$ wird $Al^{3+}$ freigesetzt, welches im Magen und im oberen Dünndarm zum Teil resorbiert wird und über das Blut sowohl in die Knochensubstanz als auch in andere Teile des Körpers (Leber, Erythrozyten etc.) gelangt und bei langjährigem Einwirken erhebliche Schädigungen hervorruft (vgl. beispielsweise Hämodialyse-Encephalopatie, Osteomalazie mit Neigung zu Spontanfrakturen, Hypercalcämie, Muskelschwäche) (A.C. Alfrey, J.M. Mishell, J. Burks, S.R. Contiguglia, H. Rudolph, E. Lewin, J.H. Holmes, Trans. Amer. Soc. Artif. Int. Org. 18, 257 (1972); A.C. Alfrey, G.R. LeGendre, W.D. Kaehny, New Engl. J. Med. 294, 184 (1976); A.M. Pierides, W.G. Edwards jr., U.X. Cullum jr., J.T. McCall, H.A. Ellis, Kidney Intern. 18, 115 (1980). Laut EDTA-Statistik sterben von den jährlich 10 bis 12% der Dialysepatienten mit zerebralen Zwischenfällen nicht wenige an einer Dialyse-Encephalopatie (H. Pogglitsch, Nieren- und Hochdruckkrankheiten 10, 210 (1981).

Die Herabsetzung der durchschnittlichen Aluminiumhydroxiddosis auf ein Drittel bewirkte einen signifikanten Abfall der Plasmaaluminiumkonzentrationen. Ein Absetzen der zur Zeit verwendeten Aluminiumpräparate zur Phosphatbindung scheint also vom medizinischen Standpunkt dringend geboten zu sein. Bis jetzt stand jedoch keine geeignete Ersatztherapie zur Verfügung.

Aus der Nahrung werden pro Tag ca. 2000 g Wasser und 2,7 g Phosphat im Darm resorbiert. Dazu kommen aus den Verdauungssäften nochmals 8000 g Wasser und 0,3 g Phosphat. Auch die Resorption von $Ca^{2+}$ und $Fe^{2+}$ erfolgt im oberen bzw. mittleren Dünndarm.

Aufgrund der gestörten Nierenfunktion bei chronischer Urämie müssen pro Behandlung generell dreimal wöchentlich u.a. folgende Substanzmengen aus dem Blut des Patienten entfernt werden: 3 bis 6 g Phosphat und 1500 bis 2000 g Wasser.

Zum Erscheinungsbild der Urämie gehört ausserdem ein Mangel an $Ca^{2+}$- und $Fe^{2+}$-Ionen. Pro Tag sollten diesen Patienten ca. 1 g Calcium und 100 bis 200 mg $Fe^{2+}$ verabreicht werden. Letzteres wird nur zu 10 bis 15% resorbiert. Nachteilig ist dabei eine momentane Überdosierung bei kurzzeitiger Zurverfügungstellung der jeweiligen Gesamtmenge sowie eine damit in Zusammenhang stehende Unverträglichkeit (Gefahr der Hypercalcämie). Ausserdem leiden die meisten Patienten mit chronischer Urämie an Darmträgheit bzw. Darmverstopfung. Solche Patienten weisen in vielen Fällen auch einen Mangel an Spurenelementen auf.

In der DE-OS 2 505 755 wird ein Mittel zum Heben des Blutcalciumgehalts bei Tieren und ein Verfahren zu seiner Herstellung beschrieben. Dieses Mittel besteht aus einer wässrigen Mischung von Calciumchlorid und einem gelbildenden Polymeren. Als Polymere werden Polyvinylalkohol oder Polyethylenglykol beschrieben. Diese Mittel können bei Menschen nicht eingesetzt werden, weil bisher nicht bewiesen ist, dass sie selbst, ihre Monomeren oder ihre Stoffwechselprodukte nichttoxisch sind.

Nierensteine, insbesondere Oxalatsteine, können u.a. dadurch gebildet werden, dass durch die Aufnahme von oxalatreicher Nahrung und Resorption des Oxalats im Darm bei der Harnbereitung hohe Oxalatkonzentrationen in der Niere auftreten und dann das Löslichkeitsprodukt von Calciumoxalat überschritten wird. Ähnliche Überlegungen gelten für die Bildung von Calciumphosphatsteinen.

In der DE-A-2 759 032 werden essentielle Metallkomplexe von bestimmten Oligo- bzw. Polygalakturonsäuren beschrieben, wobei in der angegebenen Formel als Metalle $Eisen^{+2}$, $Kupfer^{+1}$, $Kupfer^{+2}$, $Magnesium^{+2}$, $Kalium^{+1}$, $Kobalt^{+2}$, $Mangan^{+2}$, $Zink^{+2}$, $Chrom^{+3}$, $Molybdän^{+5}$, Vana-

dium $^{+4}$ und Nickel $^{+2}$ angegeben werden. Calcium wird nicht genannt.

Die bekannten Metallsalzmischungen dienen ausschliesslich zur Zufuhr von Metallionen im Organismus bzw. zum Ersatz von mangelnden Metallionen im Organismus.

In der FR-A-3068M werden Komplexe aus Polyose und dem Salz einer Hexuronsäuren beschrieben. Als Salze der Hexuronsäure können auch Calciumsalze verwendet werden. Wie aus der linken Spalte, Seite 1, dritter Absatz hervorgeht, handelt es sich hierbei um chemisch gebundene Komplexe. Erfindungsgemäss werden stattdessen Calciumsalze oder Calciummischsalze verwendet. Die aus dieser Literaturstelle bekannten Zusammensetzungen sind Polyuronide, die als Gele beschrieben werden und einen Aloe-Extrakt enthalten. Diese bekannten Mittel werden zur Behandlung von Wunden, Blutungen und Quetschungen, d.h. für die topische Anwendung verwendet. Mittel, die oral verabreicht werden können, werden in dieser Literaturstelle nicht beschrieben.

Die GB-A-1 394 741 betrifft ein Verfahren zur Herstellung eines Calciumalginatmaterials, welches gut löslich ist, indem man das Calciumalginat mit solubilisierenden Ionen versetzt. Als solubilisierende Ionen werden hier nur Natriumionen eingesetzt. Arzneimittel, welche eine Calciumsalz oder ein Calciummischsalz enthalten, werden in dieser Literaturstelle nicht beschrieben.

Die FR-A-6649M betrifft ein reines Magnesiumpektinat. Das Magnesiumpektinat wird zur Behandlung von Nierensteinen verwendet. Erfindungsgemäss werden jedoch Calciumsalze oder Calciummischsalze eingesetzt.

Der vorliegenden Erfindung liegt die Aufgabe zugrunde, ein oral verabreichbares Arzneimittel zur Verfügung zu stellen, mit dem Phosphat, insbesondere bei chronischer Urämie, aus dem Dünndarm entfernt werden kann. Erfindungsgemäss soll weiterhin ein Arzneimittel zur Verfügung gestellt werden, mit dem der Calcium- und Eisenspiegel des Blutes reguliert werden kann und mit welchem dem Patienten gegebenenfalls weitere Spurenelemente zur Verfügung gestellt werden. Erfindungsgemäss soll weiterhin ein Arzneimittel zur Verfügung gestellt werden, mit dem die Bildung von Nierensteinen vermieden wird. Das Mittel soll insbesondere Patienten verabreicht werden, von denen bekannt ist, dass sie zur Nierensteinbildung neigen.

Der vorliegenden Erfindung liegt weiterhin die Aufgabe zugrunde, Calciummischsalze sowie ein Verfahren zu ihrer Herstellung zur Verfügung zu stellen, die zur Bindung von Phosphat, zur partiellen Entwässerung und zur Ergänzung des $Ca^{2+}$- und $Fe^{2+}$-Bedarfs sowie des Bedarfs an Spurenelementen verwendet werden können.

Die erfindungsgemässen Arzneimittel sollen weder im Magen noch im Dünndarm $Al^{3+}$ freisetzen, sie dürfen keine schädlichen Nebenwirkungen hervorrufen, und die Grundsubstanz soll auf einfache Weise herzustellen sein.

Die Erfindung betrifft ein oral verabreichbares Arzneimittel zur Regulierung des Phosphat- und/oder Calciumspiegels bei chronischer Urämie und/oder zur Regulierung des Oxalat- und/oder Phosphatspiegels bei der Nierensteinprophylaxe, das dadurch gekennzeichnet ist, dass es mindestens ein Calciumsalz oder ein Calciummischsalz einer natürlichen oder chemisch modifizierten polymeren anionischen Carbonsäure und/oder eines ihrer Schwefelsäureester, wobei das Calciumsalz das 0,5- bis 5fache der stöchiometrisch erforderlichen Menge an Calciumionen enthält, die Kationen in dem Calciummischsalz aus der Gruppe Calcium, Eisen und/oder Kationen von Spurenelementen ausgewählt werden, die Summe der Kationen im Calciummischalz im Bereich des 0,5- bis 5fachen der stöchiometrisch erforderlichen Menge liegt, es die 0,45- bis 4,95fache Menge der stöchiometrisch erforderlichen Menge an Calciumionen und da 0,05- bis 2,5fache der stöchiometrisch erforderlichen Menge an Eisenionen und/oder Kationen von Spurenelementen enthält, und übliche Zusatzstoffe und/oder Trägerstoffe enthält und dass es in Form von Perlen, Tabletten, Kapseln, Pulver, Dragees oder Pillen vorliegt.

Ein bevorzugtes Arzneimittel enthält das Calciumsalz oder Calciummischsalz einer Polymannuronsäure, Polygalacturonsäure, Polyglucuronsäure, Polyguluronsäure, eines Oxidationsproduktes von Homoglykanen, eines Oxidationsproduktes von Heteroglykanen oder deren Gemische. Besonders bevorzugt sind Calciumalginat und/oder Calciumpektat.

Es ist bevorzugt, dass das Arzneimittel als Calciumsalz das stöchiometrische Calciumsalz enthält oder dass das Calcium in einer Menge im Bereich des 0,5- bis 2fachen, vorzugsweise 0,8- bis 2fachen der stöchiometrisch erforderlichen Menge vorhanden ist.

Ein weiteres bevorzugtes Arzneimittel enthält ein Calciummischsalz, in dem das Calcium in einer Menge des 0,2- bis 1,5fachen der stöchiometrisch erforderlichen Menge durch Eisenionen und/oder Kationen von Spurenelementen ersetzt ist.

Die Erfindung betrifft weiterhin die Verwendung von mindestens einem Calciumsalz oder Calciummischsalz einer natürlichen oder chemisch modifizierten polymeren anionischen Carbonsäure und/oder eines ihrer Schwefelsäureester, wobei das Calciumsalz das 0,5- bis 5fache der stöchiometrisch erforderlichen Menge an Calciumionen enthält, die Kationen in dem Calciummischsalz aus der Gruppe Calcium, Eisen und/oder Kationen von Spurenelementen ausgewählt werden, die Summe der Kationen im Calciummischsalz im Bereich des 0,5- bis 5fachen der stöchiometrisch erforderlichen Menge liegt, es die 0,45- bis 4,95fache Menge der stöchiometrisch erforderliche Menge an Calciumionen und das 0,05- bis 2,5fache der stöchiometrisch erforderlichen Menge an Eisenionen und/oder Kationen von Spurenelementen enthält, und üblichen Zusatzstoffen und/oder Trägerstoffen zur Herstellung eines oral verabreichbaren Arzneimittels zur Regulierung des Phosphat- und/oder Calciumspiegels bei chronischer Urämie und/oder zur Regulierung des Oxalat- und/oder Phosphatspiegels bei der Nierensteinprophylaxe.

Es ist bevorzugt, dass ein Calciumsalz oder Calciummischsalz einer Polymannuronsäure, Polygalacturonsäure, Polyglucuronsäure, Polyguluronsäure, eines Oxidationsprodukts von Homoglykanen, eines Oxidationsprodukts von Heteroglykanen oder deren Gemische verwendet wird.

Besonders bevorzugt werden das Calciumalginat und/oder das Calciumpektat verwendet. Die Verwendung erfolgt in Form von Perlen, Tabletten, Kapseln, Pulver, Dragees oder Pillen.

Die Erfindung betrifft weiterhin ein Calciummischalz, das dadurch gekennzeichnet ist, dass es ein Calcium-Eisen-Alginat und/oder -Pektat enthält.

Erfindungsgemäss werden die folgenden Vorteile erreicht:

1. Naturgemäss findet bei dieser Substanzgruppe keine $Al^{3+}$-Freisetzung (wie aus $Al(OH)_3$ in Magen und Dünndarm bzw. aus bereits gebildetem $AlPO_4$ im Dünndarm bei Verwendung der derzeitigen Phosphatbinder) statt.

2. Es wird keine Phosphat-Depletion wie bei einer $Al(OH)_3$-Medikation beobachtet.

3. Die Grundsubstanzen sind, wie Alginsäure, Pektinsäure u.a. pflanzlichen Ursprungs und bereits lebensmittelrechtlich als Zusatzstoffe zugelassen. Sie geben keine schädlichen Momoneren ab, wie dies zum Beispiel bei voll- und eventuell auch halbsynthetischen Ionenaustauschern oder anderen Polymeren der Fall sein kann.

4. Die Substanzen liegen im Dünndarm als Gele vor und sind, ebenso wie die freigesetzten Polymeren (zum Beispiel Alginsäure) und ihre monomeren Bausteine, wie Mannuronsäure, Galacturonsäure etc., biologisch durch Darmmikroorganismen vollständig abbaubar.

5. Der Bedarf an Calciumalginat beträgt zum Beispiel bei der Entfernung von 5 g Phosphat zwischen 2 und 30 g und ist damit relativ gering. Zusätzlich kann ein Überschuss des vollständig oder partiell getrockneten Mittels zur Quellzwekken verabreicht werden.

6. Die Gelkonsistenz kann entsprechend der Mengenverhältnisse der Ausgangskomponenten in weiten Grenzen bestimmt und verändert werden. Damit kann für die Verabreichung, zum Beispiel als Lyophilisat, Einfluss auf die Calciumzugänglichkeit genommen werden und damit auf die Reaktionsgeschwindigkeit der Hydroxylapatitbildung und die eventuelle Resorption zusätzlicher $Ca^{2+}$- oder $Fe^{2+}$-Ionen. Bei geringerer Calciumkonzentration im Reaktionsansatz werden weichere Gele erhalten.

7. Eine Dotierung mit erwünschten Ionen, wie $Ca^{2+}$ und $Fe^{2+}$, kann in weiten Grenzen bereits bei der Gelherstellung und durch geeignete Waschprozesse vorgenommen werden. Es treten also keine Calcium- und Magnesiumverluste wie bei einer $Al(OH)_3$-Medikation ein, sondern der Urämiker kann mangelnde Ionen durch das neue Verfahren ergänzt bekommen.

8. Die Konsistenz von Gelen, in denen 50% des $Ca^{2+}$ durch $Fe^{2+}$ ersetzt wurden, entspricht der von reinen Calciumalginatgelen.

9. Das Gelmaterial ist bei Abwesenheit von Phosphat oder Oxalat unter physiologischen pH-Verhältnissen stabil.

10. Durch langsame Lösung des Gels in Gegenwart von Phosphat und die sofortige Bildung von Hydroxylapatit (HAP) – $Ca_{10}(PO_4)_6(OH)_2$ – wird auch bei Dotierung des Gels mit einem Calciumüberschuss die Gefahr der Hypercalcämie vermieden. Letztere wurde bei Calciumverabreichung in Form von $CaCl_2$ (sauer), Ca-Lactat, $CaCO_3$ (basisch, $CO_2$-Entwicklung) und Ca-Acetat beobachtet.

11. Bei geeigneter Auslegung des Gels ist nach einer Startphase eine gleichmässige Bildung von Hydroxylapatit und Ca-Resorption zu erwarten. Bei Gelen mit Ca-Überschuss kann sofort eine partielle Fällung von HAP eintreten, und erst dann sorgt eine langsame Gelauflösung für die Freisetzung von $Ca^{2+}$ für die erwünschte Resorption beim Urämiker.

12. Bei fünfmal gewaschenen Gelen sind nach ca. drei Minuten 50% des angebotenen Phosphats bereits umgesetzt (vgl. Figur 1). Lyophilisate dieses Materials reagieren noch schneller, da dort die Gelstruktur aufgebrochen ist, was zu einer leichteren Zugänglichkeit des Calciums führt. Pulverisieren würde diesen Effekt noch steigern. Bei der Herstellung des Ausgangsmaterials lässt sich jedoch auch für ein zu lyophilisierendes Material die geeignete Reaktionsgeschwindikgeit optimieren.

13. Ausgefälltes HAP wird mit dem Darminhalt entfernt, analoges gilt für Oxalat als Calciumoxalat. Bei niedrigeren pH-Werten im Enddarm kann eine partielle Auflösung von HAP erfolgen, jedoch werden weder $Ca^{2+}$ noch Phosphat in diesem Darmabschnitt resorbiert.

14. Auch in Gegenwart von Phosphat ist trotz der geringeren Löslichkeit des HAP eine partielle Sulfatabtrennung möglich.

15. Verabreichungsformen können sein: Gel, partiell oder vollständig getrocknetes Gel (Perlen) und Lyophilisat (evtl. auch als Pulver). Zur Vermeidung einer Aufnahme von Wasser aus dem Magen durch Aufquellen wäre jedoch eine magensaftresistente Kapselung erforderlich.

16. Bei Verabreichung von getrocknetem Calciumalginatgel oder Calciumpektatgel ist eine Anwendung als Quellsubstanz möglich (5 bis 10 g $H_2O$/g) Trockenform).

17. Das in Gegenwart von zum Beispiel Natriumphosphat aus dem Gel freigesetzte $Ca^{2+}$ reagiert bevorzugt mit Oxalationen zu schwer löslichem Calciumoxalat und verhindert so die Oxalatresorption aus dem Darm in das Blut. Durch die damit verringerte Oxalatkonzentration des Blutes wird die existierende Gefahr der Oxalatsteinbildung während der Bereitung des Endharns infolge des Konzentrierungsprozesses in der Niere und der resultierenden Überschreitung des Löslichkeitsprodukts von Ca-Oxalat weitgehend eingeschränkt bzw. aufgehoben.

Erfindungsgemäss werden die Calciumsalze natürlicher oder chemisch modifizierter polymerer, anionischer Carbonsäuren oder Schwefelsäureester verwendet. Solche natürliche polymere, anionische Carbonsäuren, wie sie erfindungsgemäss eingesetzt werden, sind in vielen Fällen Kationenaustauscher, die mit $Ca^{2+}$ Gele bilden. Erfindungsgemäss können Verbindungen der folgenden Gruppen A, B und C verwendet werden:

A) Polyuronide und verwandte Substanzen mit relativ hohen Anteilen an Uronsäuren, wie beispielsweise Glucuronsäure, Galacturonsäure, Guluronsäure, Mannuronsäure, Iduronsäure. Die in der Natur gefundenen Alginsäuren, die zwischen 0 und 100% Mannuronsäure und 0 bis 70% Guluronsäure enthalten, können ebenfalls verwendet werden. Besonders geeignet sind:

1. Polymannuronsäure, wie zum Beispiel Alginsäure (Alginsäure wird erfindungsgemäss besonders bevorzugt verwendet.)
2. Polygalacturonsäure, wie zum Beispeil Pektinsäure oder Pektine mit niedriger Methanolveresterung
3. Polyglucuronsäure, beispielsweise solche, die durch mikrobielle Oxidation von Cellulose hergestellt wird,
4. Polyguluronsäure, beispielsweise bestimmte Alginsäuren

Selbstverständlich können auch Gemische der genannten Polyuronide eingesetzt werden.

B) Oxidationsprodukte von Homoglykanen, wie zum Beispiel Agarose, Dextrane, Cellulose, Pullulane usw.

C) Oxidationsprodukte von Heteroglykanen:

1. Galactomannane (Guar Gum = Guaran; Carboxy-Guar Gum) (Carob-Gum = Locust Bean Gum = Johannisbrotkernmehl)
2. sulfatveresterte Carrageenane (kappa, i)
3. Glucomannane

sowie Mischungen dieser Verbindungen

D) Mischungen der unter A bis C aufgeführten Stoffgruppen

Im folgenden sind einige Strukturformeln dieser Verbindungen aufgeführt.

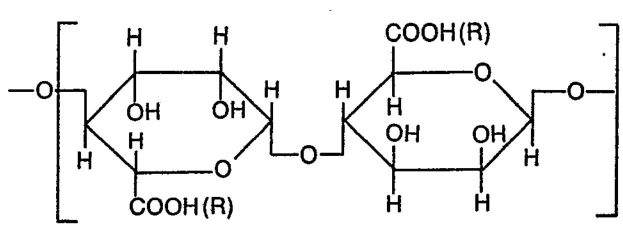

Alginsäure

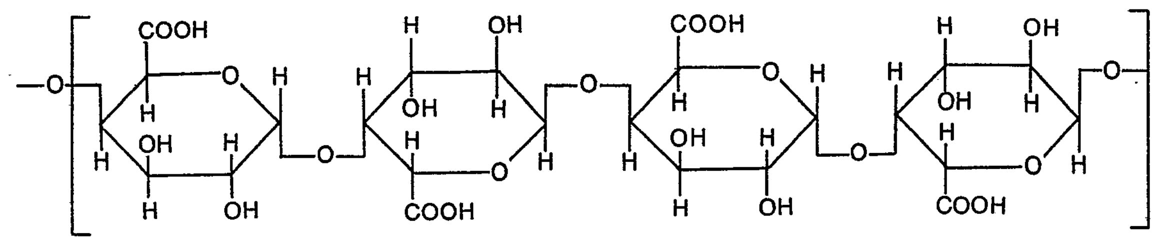

Pektinsäure (Poly-D-Galacturonsäure)

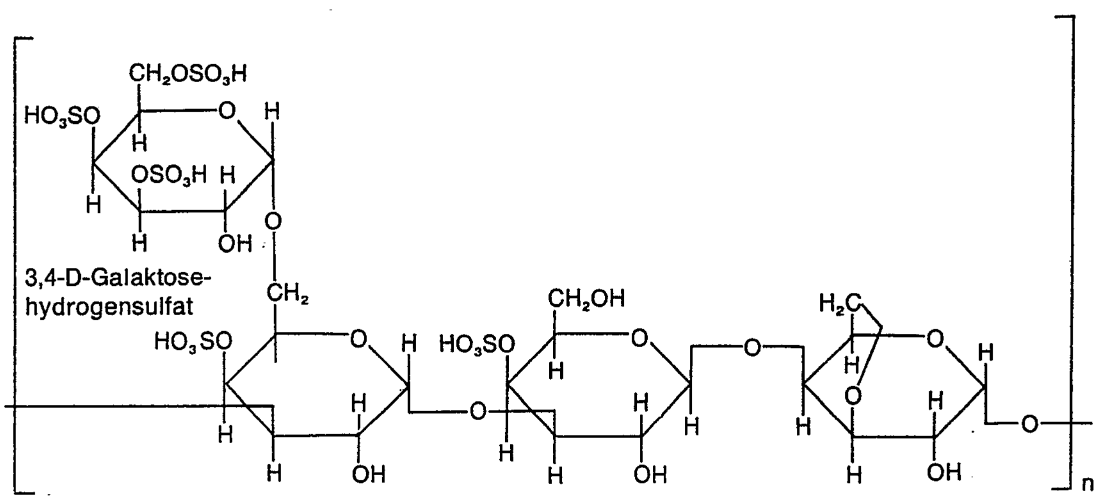

3,4-D-Galaktose-hydrogensulfat

D-Galactose-4-sulfat ϰ-Carrageen 3,6-Anhydro-D-galactose

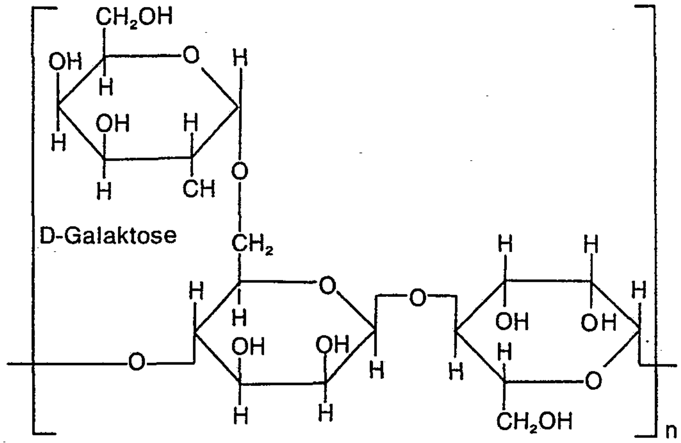

D-Galaktose

D-Mannose D-Mannose

Guaran

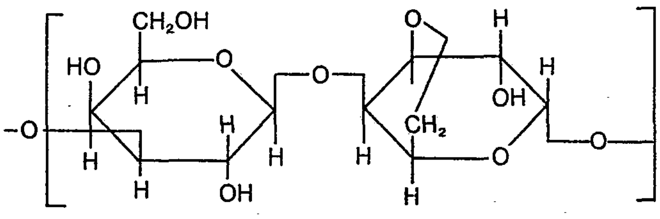

D-Galaktose 3,6-Anhydro-L-galaktose

Agar-Agar

Die Oxidation der Verbindungen der Gruppen B und C ist bekannt und wird in der Literatur beschrieben (P. Nuhn, Chemie der Naturstoffe, Akademie-Verlag, Berlin (1981), S. 166, 167). Aldosen, besonders in Polymeren, können ohne Spaltung von C-C-Bindungen durch zum Beispielen Sauerstoff in Gegenwart von Platin, Permanganat oder Stickstoffdioxid an ihren endständigen freien primären Hydroxylgruppen schonend oxidiert werden, wobei entsprechende Uronsäuren (Carboxylfunktionen) gebildet werden, sofern die endständige Position nicht am Aufbau des Glykans beteiligt war. Der bei Monosacchariden notwendige Schutz der Aldehydfunktion (anomeres Zentrum) ist in polymeren Zuckern bis auf endständige Einheiten meistens gegeben. Bei Hexosen sind auch Epimerisierungen am C-5 leicht möglich.

Bevorzugt verwendet man bei dem erfindungsgemässen Verfahren Polysaccharide. Diese gehören zur Stoffklasse der Kohlenhydrate und sind hochmolekulare Verbindungen, die aus glykosidisch verknüpften Monosaccharideinheiten aufgebaut sind. Polysaccharide bilden mit Wasser hochviskose Lösungen oder in Gegenwart von $Ca^{2+}$- und anderen Ionen Gele und finden als Verdikkungs- und Geliermittel für Lebensmittel sowie in der Pharmazie als Träger- und Hüllsubstanzen für Medikamente Verwendung. Derartige Polysaccharide sind für die vorliegende Erfindung besonders gut geeignet, und im folgenden werden einige Substanzen näher erläutert, die erfindungsgemäss besonders bevorzugt verwendet werden (vgl. auch Beitrag «Polysaccharide» in Enzyklopädie Naturwissenschaft und Technik, S. 3409–3412, Verlag Moderne Industrie, Landsberg/Lech (1980)).

Alginsäure und ihre Salze

Polymerisat aus D-Mannuronsäure und meist wenig (jedoch bis 70% gefunden) L-Guluronsäure; β-1,4-glykosidisch verknüpft; MG 12 000 bis 200 000 Dalton; Quelle: Braunalgen; leicht verdaulich, bis 1000 COOH-Gruppen im Molekül

Alginsäure und ihre Salze wurden bisher bei der Herstellung von Speiseeis, Marmeladen, Fruchtgelees, Kosmetika, Seifen, Cremes, essbaren Wursthüllen, Mayonnaisen, Pudding, Fertigsuppen, Suppenwürfeln und als Schlankheitsmittel eingesetzt. Sie sind erfindungsgemäss besonders bevorzugt, da es sich um nichttoxische Substanzen handelt und lebensmittelrechtlich oder arzneimittelrechtlich keinerlei Bedenken bei ihrer Anwendung bestehen. In Ca-Alginat-Gelen können selbst Erythrozyten für längere Zeit in funktionsfähigem Zustand aufbewahrt werden (G. Pilwat, P. Washausen, J. Klein, U. Zimmermann, Z. Naturforsch. 35c, 352 (1980)).

Pektinsäure und ihre Salze (Pektate)

Pektinsäure besteht aus α-1,4-glykosidisch verbundenen Galacturonsäureeinheiten. Im Pektin (Salze: Pektinate) sind 20 bis 60% der Säuregruppen mit Methanol verestert. Sie haben einen geringen Gehatl an Galactose und Arabinose. Pektinsäure ist daraus durch Verseifung erhältlich. Citrus-Pektin stellt reine Polygalacturonsäure dar.

Die Molekulargewichte liegen zwischen 30 000 und 500 000 Dalton. Ca-Pektat ist in Wasser unlöslich; Pektinsäure besitzt eine Selektivität für Calcium.

Pektinate werden in zuckerarmen oder zuckerfreien Gelees, Marmeladen, Milchpudding, Fisch- und Fleischwaren in Gelee oder Aspik, zur Farberhaltung bei gefrorenen Erdbeeren, Kosmetika, Arzneimitteln, Trockenmitteln und Schutzkolloiden, Speiseeiszusatz, Kaugummi und in der Schmelzkäsezubereitung verwendet. Gegen ihren Einsatz bestehen somit ebenfalls keine lebensmittelrechtlichen oder arzneimittelrechtlichen Bedenken.

Agarose

Agarose besteht aus abwechselnden Einheiten von β-1,3-verknüpfter D-Galactose und α-1,4-verknüpfter 3,6-Anhydro-L-Galactose. Ein kleiner Teil der Galactosemoleküle ist an C-6 methyliert oder mit Schwefelsäure verestert.

Dextrane

Dextrane stellen α-1,6- und α-1,4- (α-1,3-) verknüpfte Polyglucose dar, MG bis mehrere Millionen Dalton. Sephadex ist mit Epichlorhydrin dreidimensional vernetztes Dextran, als solches indifferent gegen Anionen und Kationen, Gelbildner aufgrund zahlreicher OH-Gruppen; verwendet als Matrix für funktionelle Gruppen in Ionenaustauschern.

Carrageen(ane)

Carrageenane mit MG von 100 000 bis 800 000 Dalton sind bekannt; ca. 25% Sulfatgehalt; verschiedene Typen: kappa-Carrageenan: aus alternierenden 1,3- bzw. 1,6-verknüpfter 3,6-Anhydro-α-D-Galactose und D-Galactose-4-sulfat; gelbildend, nur noch teilweise oxidierbar lambda-Carrageenan: α-1,3-glykosidisch verbundene D-Galactose-2(4)-sulfat-Reste und 1,4-verbundene D-Galactose-2,6-disulfat-Reste, geliert nicht, noch partiell an C-6 oxidierbar

i-Carrageenan: aus D-Galactose-4-sulfat in 1,3-Bindung und 3,6-Anhydro-α-D-Galactose-2-sulfat in 1,4-Bindung aufgebaut, bildet mit $Ca^{2+}$ Gele.

Johannisbrotkernmehl (Carubin) / Guarmehl = Guaran = Guargummi

Galactomannane. Die letztgenannten Substanzen werden zum Emulgieren, zum Stabilisieren, Dicken und Binden von Speiseeis, Weichkäse, Backwaren und Sossen eingesetzt, und es gibt keinerlei Mengenbegrenzung hinsichtlich ihrer Verwendung (vgl. J. Schormüller, Lehrbuch der Lebensmittelchemie, 2. Auflage 1974, S. 305, 100–109, Springer-Verlag, Berlin, Heidelberg, New York).

Die Calciumsalze oder Calciummischsalze der genannten Verbindungen können in stöchiometrischer oder in nichtstöchiometrischer Form vorliegen. Beispielsweise kann das Calcium in den Calciumsalzen in einer Menge im Bereich der 0,5- bis 5fachen, bevorzugt der 0,6- bis 2,8fachen, mehr bevorzugt der 1- bis 2fachen, besonders bevorzugt der 1,5fachen, stöchiometrisch erforderlichen Menge vorhanden sein.

Es wurde weiterhin gefunden, dass in den zur Gelhärtung verwendeten, zum Beispiel 0,3- bis 0,8M, vorzugsweise 0,5M Calciumsalzlösungen 0,05 bis 0,5M der Calciumionen durch Eisenionen und/oder andere Kationen, insbesondere Kationen von Spurenelementen, ersetzt sein können. Mit anderen Worten können die Calciumgele mit Eisen und anderen Kationen dotiert sein.

In den Calciummischsalzen ist das Calcium in einer Menge die der 0,45- bis 4,95fachen, vorzugsweise der 0,6- bis 2,8fachen, besonders bevorzugt der 1- bis 2fachen, der stöchiometrisch erforderlichen Menge entspricht, vorhanden. In der folgenden Tabelle I sind die jeweiligen Konzentrationen der einzelnen Kationen, bezogen auf die stöchiometrisch erforderliche Menge, angegeben.

Tabelle I

Calciummischsalze

| Bereich | Summe der Kationen | Ca-Ionen | Fe-Ionen, Spurenelemente |
|---|---|---|---|
| allgemein | 0,5–5 | 0,45–4,95 | 0,05–2,5 |
| bevorzugt | 0,8–3 | 0,6–2,8 | 0,2–1,5 |
| besonders bevorzugt | 1,3–2,5 | 1 –2 | 0,3–0,6 |

Als Beispiele sind in der folgenden Tabelle II die stöchiometrisch erforderlichen Mengen an verschiedenen Metallen, bezogen auf Alginsäure, aufgeführt.

Tabelle II

| Stöchiometrische Mengen | | | |
|---|---|---|---|
| $Ca^{2+}$ | 104 mg/g | als | Trockenperlen |
| $Fe^{3+}$ | 98 mg/g | als | Trockenperlen |
| $Fe^{2+}$ | 130 mg/g | als | Trockenperlen |
| $Na^{+}$ | 118 mg/g | als | Trockenperlen |
| $K^{+}$ | 185 mg/g | als | Trockenperlen |

Besonders bevorzugte Substanzen sind die folgenden:

1. für die Phosphatelimination
   Ca-Alginat
   Ca-Fe-Alginat
   Ca-Pektat
   Ca-Fe-Pektat
   Fe(II)-Ca-Alginat
   Fe(III)-Ca-Alginat

2. für die Oxalatelimination
   Ca-Alginat
   Ca-Pektat

Gemäss einer besonders bevorzugten Ausführungsform enthalten die oben genannten Substanzen die Calciumionen in der 1- bis 2fachen der stöchiometrisch erforderlichen Menge und die anderen Ionen in der 0,3- bis 0,6fachen der stöchiometrisch erforderlichen Menge.

Besonders bevorzugte erfindungsgemässe Substanzen besitzen die folgende Zusammensetzung:

| | $Ca^{2+}$ | Fe | $K^+$ | $Na^+$ | Σ |
|---|---|---|---|---|---|
| IGB-21 | 160 | – | 8 | 20 | 188 mg/g |
| IGB-27 | 140 | 45 | 8 | 20 | 213 mg/g |

Die erfindungsgemässen Mischsalze können auf einfache Weise hergestellt werden. Man verwendet die wässrige Lösung der Polycarbonsäuren oder Schwefelsäureester, wobei diese wässrige Lösung eine Konzentration von 2 bis 20%, vorzugsweise von 4 bis 12%, besonders bevorzugt von 6 bis 10%, besitzt. Der pH-Wert der wässrigen Lösung wird mit einem Alkalihydroxid, wie beispielsweise Natriumhydroxid, auf einen Wert im Bereich von 5 bis 8, vorzugsweise 7,4, eingestellt. Die Lösung der Polycarbonsäure oder des Schwefelsäureesters kann man dann gegebenenfalls 1 bis 2 Stunden bei Zimmertemperatur rühren. Diese Lösung gibt man bei einer Temperatur im Bereich von 0 bis 100 °C, vorzugsweise im Bereich von 2 bis 50 °C, besonders bevorzugt bei 10 bis 30 °C, in wässrige Lösungen von 0,2 bis 2M, vorzugsweise 0,3 bis 1M, besonders bevorzugt 0,5M, von Calciumsalzen, deren pH-Wert mit Alkalihydroxid auf einen Wert im Bereich von 2 bis 8 eingestellt wurde. Ein Teil der Calciumionen kann durch $Fe^{2+}$ oder andere Kationen ganz oder teilweise ersetzt werden.

Bevorzugt verwendet man bei der Zugabe der verschiedenen Kationen die Chloride, da Chloride physiologische Anionen darstellen, die beispielsweise im Blut vorkommen und als Salzsäure den wesentlichen Anteil der Magensäure stellen. Es können aber auch andere Salze der Metalle, die im Körper keine Giftwirkung ausüben, wie beispielsweise anorganische Salze, wie Carbonat, oder organische Salze, wie die Acetate, Ascorbate, Citrate, gluconate, Lactate, Levulinate, Malonate, Pantothenate, Saccharate oder Tartrate, verwendet werden.

Das erhaltene Reaktionsprodukt, das im allgemeinen in Form von Perlen ausfällt, wird dann abfiltriert und die gewünschte Konzentration an ungebundenen Kationen durch einen für den jeweiligen Anwendungsfall zu standardisierenden Waschprozess eingestellt. Die Kationenkonzentrationen werden komplexometrisch bestimmt.

Der erhaltene Niederschlag kann nach dem Auswaschen getrocknet werden. Das Trocknen erfolgt nach an sich bekannten Verfahren. Der Wassergehalt im Endprodukt kann im Bereich von 0 bis 50% liegen.

Das erhaltene Produkt kann dann in an sich bekannter Weise zu Pillen, Dragees, Kapseln oder Tabletten verarbeitet werden. Die täglich zu verabreichende Dosis liegt je nach Gelzusammensetzung und Dotierung zwischen 2 und 30 g Trockenmasse und hängt ausserdem vom pH-Wert des Phosphat resorbierenden Darmabschnitts, von der Menge, der Zusammensetzung und der Verweilzeit der Nahrung im Darm ab. Fig. 2 zeigt die pH-Abhängigkeit der möglichen Phosphatbindung. Der Einfluss verschiedener Phosphat-Gel-Verhältnisse auf die Phosphatbindungskinetik ist in Figur 3 dargestellt.

Die folgenden Beispiele erläutern die Erfindung.

In den Beispielen wurden die folgenden Bestimmungsverfahren verwendet:

Bestimmungsmethoden

1) Bestimmung von $Ca^{2+}$:

Zur Bestimmung des Calciumgehalts der Calciumalginat- bzw. Calciumpektatgele wurden diese bei pH 8 in 0,1 Mol/l EDTA gelöst. Überschüssiges EDTA wurde mit einer 0,1molaren $MgCl_2$-Lösung bei pH 10 zurücktitriert (Eriochromschwarz-T-Mischindikator).

2) Bestimmung von $HPO_4^{2-}$:

$HPO_4^{2-}$ wurde nach der Methode von Gomorri (G. Gomorri, J. Lab. Clin. Med. 27, 955 (1942)) photometrisch bestimmt. Dabei bildet anorganisches Phosphat mit Natriummolybdat Phosphormolybdat, das durch Reduktion mit p-Methylaminophenolsulfat in kolloidales Molybdänblau überführt wird, welches photometrisch bestimmt wird.

3) Bestimmung von Eisen ($Fe^{2+}$ plus $Fe^{3+}$) in Calcium-Eisen-Alginat

1 g Calcium-Eisen-Alginat (IGB-27) wird mit 5 ml konz. $HNO_3$ angelöst und das Gesamteisen als $Fe^{3+}$ durch Titration mit 0,1 M Titriplex III bei pH 2,5 (Indikator 5-Sulfosalicylsäure; Umschlag von blau nach farblos) bestimmt.

1 ml 0,1 M Titriplex-III-Lösung = 5,585 mg Eisen. 1 g IGB-27 enthält 45,2 mg Eisen.

4) Bestimmung von Oxalat

Die titrimetrische Bestimmung von Oxalat erfolgte mit einer 0,02 M Kaliumpermanganat-Masslösung, die zur Stabilisierung 1 Stunde lang gekocht worden war. 10 ml der Probe (vgl. Beispiel 11) wurden mit 150 ml bidestilliertem Wasser und 10 ml einer 1+4 verdünnten konzentrierten Schwefelsäure versetzt, auf 75 bis 85 °C erhitzt und bis zu einer bleibenden schwachen Rosafärbung mit der $KMnO_4$-Lösung titriert.

5) Bestimmung von Natrium und Kalium

Die K- und Na-Bestimmungen erfolgten nach Auflösung der Gele in EDTA (Titriplex-III)-Lösung durch Flammenphotometrie oder Atomabsorption nach Standardverfahren.

Beispiel 1

Darstellung eines Calciumalginatgels (hier 8%) (IGB-11, IGB-21)

8 g Alginsäure (Sigma Typ III aus Kelp) werden unter Rühren in 80 ml Wasser gelöst und mit 1M NaOH auf pH 7,4 gebracht. Alternativ kann eine derartige Lösung auch durch Lösen von Natriumalginat (Protanal LFR 5/60 der Fa. Protan, Norwegen) hergestellt werden. Die klare Natriumalginatlösung wird mit Wasser auf 100 ml aufgefüllt, 2 Stunden bei Raumtemperatur gerührt und 12 Stunden so belassen. Die Na-Alginat-Lösung wird zur Härtung in 500 ml einer 0,4M $CaCl_2$-Lösung eingetropft. Beim Eintropfen bilden sich Calciumalginatperlen mit einem Durchmesser von ca. 5 mm, die in 12 Stunden bei 4 °C vollständig durchhärten.

Auswaschen nicht gebundener Ca-Salze (u. a. Salze, falls zugesetzt):

Die wie oben hergestellten Ca-Alginat-Perlen enthalten neben den an den Carboxylgruppen der Alginsäure gebundenen Calciumionen noch freie, auswaschbare Calciumsalze. Diese können dadurch entfernt werden, dass die Perlen mit dem 3-fachen Volumen Wasser gewaschen werden (IGB-11, IGB-21).

Trocknen der Calciumalginatperlen:

Die Ca-Alginat-Perlen werden gefriergetrocknet, alternativ kann 24 Std. bei 40 °C getrocknet oder zur Vermeidung des Aufbrechens der Gelstruktur nur partiell entwässert oder mit strukturerhaltenden Zusätzen gearbeitet werden:

Gehaltsbestimmungen (IGB-21):

Ca: 160 mg/g; K: 8,2 mg/g; Na: 20 mg/g Trockenperlen.

Dotierung von Calciumalginatgelen mit $Fe^{2+}$ (u. a.):

$Fe^{2+}$ und andere Spurenelemente können in Calciumalginat eingebracht werden, indem die oben beschriebene Natriumalginatlösung mit einer 0,4M $CaCl_2$-Lösung gehärtet wird, der $Fe^{2+}$ oder andere Salze nach Erfordernis von 0,01 bis 0,5M im gleichen Volumen zugesetzt wird. Die erforderliche bzw. gewünschte Dotierung ist in weiten Grenzen einstellbar, ebenso wie die zu erzielende Gelkonsistenz vor einer nachfolgenden Trocknung. Die Intensität des nachgeschalteten Waschprozesses entscheidet über den adsorptiv gebundenen Anteil von $Ca^{2+}$, $Fe^{2+}$ und anderer Salze.

Beispiel 2

Darstellung eines Calciumpektatgels (hier 15%)

15 g Pektinsäure (Serva Nr. 31 680) werden unter Rühren in 80 ml Wasser gelöst und mit 2M NaOH auf pH 7,4 gebracht. Die hochviskose Lösung wird mit Wasser auf 100 ml aufgefüllt und 2 Stunden bei 60 °C gerührt. Diese Natriumpektatlösung wird zur Härtung in 500 ml einer 1M $CaCl_2$-Lösung eingetropft, die mit 1M NaOH auf pH 7,4 eingestellt wurde. Beim Eintropfen bilden sich Calciumpektatperlen mit einem Durchmesser von ca. 5 mm, die in 15 Stunden bei 4 °C vollständig durchhärten. Der Gehalt an freien, nicht an Carboxylgruppen gebundenen Calciumsalzen kann durch Waschprozesse eingestellt werden. Die Calciumpektatgele können, wie für Calciumalginatgele beschrieben, getrocknet und mit $Fe^{2+}$, $Fe^{3+}$ und Spurensalzen dotiert werden.

Beispiel 3

Darstellung eines Eisen-Calcium-Alginatgels (IGB-27)

8 g Natriumalginat (Protanal) werden unter Rühren in 80 ml bidestilliertem Wasser gelöst, die Lösung mit 1M NaOH auf pH 7,4 gebracht und mit bidestilliertem Wasser auf 100 ml aufgefüllt.

Diese 8%ige Na-Alginatlösung wird zur Härtung in 500 ml einer Lösung, die 0,25 M an $FeCl_2$ und 0,4 M an $CaCl_2$ ist, getropft. Beim Eintropfen bilden sich Perlen von Eisen-Calcium-Alginat, die bei einer Temperatur von 4 °C innerhalb von 12 Stunden vollständig durchhärten.

Zur Entfernung nicht stöchiometrisch gebundener Eisen- und Calciumionen aus den Perlen wird – wie in Beispiel 1 beschrieben – mit Wasser gewaschen. Die Trocknung benötigt bei 40 °C ca. 24 Stunden.

Gehaltsbestimmungen:

Ca. 140 mg/g; Fe: 45,2 mg/g; K: 7,8 mg/g; Na: 20 mg/g Trockenperlen.

Beispiel 4

Darstellung von Eisen(III)-Calcium-Alginatgel bei pH 2 (IGB-22)

Eine 8%ige Natrium-Alginatlösung (wie in Beispiel 1 hergestellt) lässt man in eine Härtungslösung eintropfen, die durch Lösen von 0,125M Eisen(III)-Citrat in einer erhitzten 0,5 M $CaCl_2$ bei pH 2 erhalten wurde.

Härtung, Waschung und Trocknung wie in Beispiel 3 beschrieben. Die Perlen besitzen eine dunkelviolette Färbung.

Beispiel 5

Darstellung von Eisen(III)-Calcium-Alginatgel bei pH 6,7 (IGB-23)

66 g Eisen(III)-Citrat werden unter Erhitzen in 100 ml bidestilliertem Wasser gelöst und mit 1M NaOH auf pH 6,7 eingestellt. Unter Einbeziehung dieser Lösung werden in üblicher Weise 2 Liter einer 8%igen Na-Alginatlösung hergestellt, die damit 0,125M an Eisen(III)-Citrat ist. Diese Eisen(III)-Citrat/Na-Alginat-Mischung tropft man in eine 0,4 M $CaCl_2$-Lösung ein. Die dunkelvioletten Kugeln werden in bereits beschriebener Weise gehärtet, gewaschen und getrocknet.

Beispiel 6

Darstellung von Eisen(II)-Calcium-Alginatgelen bei pH 2,6 (IGB-25) und pH 6,8 (IGB-26)

Eine 8%ige Lösung von Natriumalginat wird analog Beispiel 1 hergestellt (100 ml). Die Härtungslösung (500 ml) hat folgende Zusammensetzung: 0,25 M $FeCl_2$, 0,4 M $CaCl_2$ und 0,25 M L-Ascorbinsäure (Vitamin C).

a) Zu 250 ml der Härtungslösung mit pH = 2,6 gibt man tropfenweise 50 ml der Na-Alginatlösung,
b) zu weiteren 250 ml der mit 1 M NaOH auf pH 6,8 eingestellten Härtungslösung (violett gefärbt) gibt man tropfenweise 50 ml der Na-Alginatlösung.

Nach 12 Stunden Härtungszeit werden die entstandenen Perlen wie üblich gewaschen und getrocknet (vgl. Beispiel 3). Die Präparate sind wie folgt gefärbt: IGB-25 bräunlichgelb; IGB-26 dunkelviolett.

Beispiel 7
In vitro Phosphatbindungskapazität verschiedener Substanzgruppen auf $Al(OH)_3$- bzw. Ca-Alginat-Basis (vgl. Figur 4)

Jeweils 1 Tablette bzw. 1 Kapsel der handelsüblichen Phosphatbinder auf $Al(OH)_3$-Basis &Aludrox, Antiphosphat und Phosphonorm) sowie je 0,45 g trockenes nichtgewaschenes Ca-Alginat (IGB-15), trockenes gewaschenes Ca-Alginat (IGB-11) und frisch gefälltes $Fe(OH)_3$ (IGB-31) wurden zur Simulierung der Magenpassage in jeweils 100 ml Tris-Puffer mit 0,48 g $K_2HPO_4$(≙ 0,2 g Phosphat) bei pH 2 und 37 °C für 1 Stunde auf dem Rollermixer inkubiert (1. Probenahme für Phosphatbestimmung).

Danach wurde mit NaOH auf pH 8 eingestellt, weitere 0,48 g $K_2HPO_4$ zugesetzt und 1 Stunden inkubiert («Darmpassage») (darauf 2. Probe für Phosphatbestimmung entnommen). Nach Einstellung auf pH 7,5 wurde eine weitere Stunde inkubiert (3. Probenahme), danach bei pH 7,0 analog verfahren (4. Probe für Phosphatbestimmung).

Die Ergebnisse zeigt Figur 4. Von den insgesamt vorgelegten 25 mM Phosphat werden nur von Phosphonorm unter diesen Bedingungen mehr als 50% gebunden. Die auf Polyuronsäuren basierenden Präparationen sind jedoch besser geeignet als die weiteren Al-haltigen Präparate.

Die in-vitro-Phosphatbindungskapazität je Gramm der Phosphatbinder Aludrox, Antiphosphat, Phosphonorm und Ca-Alignat (IGB-11) ist in Tabelle I gegenübergestellt.

Tabelle I

| | Aludrox 1 Tabl. | Antiphosphat 1 Tabl. | Phosphonorm 1 Kapsel | Ca-Alginat gewaschen (IGB-11) 450 mg |
|---|---|---|---|---|
| Phosphatbindung | 0,096 g/ Tablette | 0,09 g/ Tablette | 0,25 g/ Kapsel | 0,155 g |
| Gewicht | 5800 mg | 790 mg | 400 mg | 450 mg |
| Phosphatbindungskapazität pro Gramm | 0,17 g $PO_4^{3-}$ | 0,114 g $PO_4^{3}$↗ | 0,625 g $PO_4^{3-}$ | 0,34 g $PO_4^{3-}$ |

Beispiel 8
Phosphatbindungskapazität verschiedener Alginat-Präparationen im Vergleich mit Aludrox und $Fe(OH)_3$

Je 1 g der folgenden Substanzen wurden in 200 ml Tris-Puffer aufeinanderfolgend bei verschiedenen pH-Werten (pH 2, pH 8, pH 7,5, pH 7) mit 0,4 g Phosphat für je 1 Std. inkubiert und anschliessend die Menge an freiem Phosphat bestimmt:

IGB-11: Ca-Alginat (Sigma), gewaschen
IGB-15: Ca-Alginat (Sigma), nicht gewaschen
IGB-21: Ca-Alginat (Protan), gewaschen
IGB-22: Eisen(III)-Ca-Alginat, pH 2 (Beispiel 4)
IGB-23: Eisen(III)-Ca-Alginat, pH 6,7 (Beispiel 5)
IGB-24: Eisen(II)-Ca-Alginat (ohne Beispiel)
IGB-25: Eisen(II)-Ca-Alginat, pH 2,6 (Beispiel 6)
IGB-26: Eisen(II)-Ca-Alginat, pH 6,8 (Beispiel 6)
IGB-27: Eisen(II/III)-Ca-Alginat (Beispiel 3)
IGB-31: frisch gefälltes $Fe(OH)_3$
Aludrox.

Die Ergebnisse zeigt Figur 5:

Unter sauren Bedingungen («Magensaft») reagieren reine Ca-Alginate nicht mit Phosphat, während das Präparat IGB-27 dort bereits 16% seiner Bindungskapazität zeigt. Unter neutralen bzw. leicht alkalischen Bedingungen («Darmpassage») vermag IGB-21 ca. 50% Phosphat zu eliminieren, IGB-27 sogar 73%. Aludrox bindet unter diesen Bedingungen nur 24% des vorgelegten Phosphats.

Beispiel 9
Phosphatelimination aus menschlichem Duodenalsaft

500 ml Duodenalsaft (pH 7–8) wurden mit 2 g Trinatiumphosphat ($Na_3PO_4$) angereichert und bei 37 °C unter Rühren mit 2,4 g Calciumalginatperlen (IGB-21) über 2 Stunden inkubiert. Während der ersten 45 Minuten trat eine deutliche Phosphatabnahme ein, danach war die Verminderung der Phosphatkonzentration nur noch gering (Figur 6). Nach 120 Minuten befand sich die Phosphatkonzentration wieder in der Nähe des Ausgangswerts vor der Beladung.

Beispiel 10
In-vivo-Phosphatelimination

10 Patienten mit chronischem Nierenversagen, von denen 9 hämodialysiert wurden, und die trotz der konventionellen Therapie mit Aluminiumhydroxid zu hohe Serum-Phosphatspiegel aufwiesen, wurden mit Calciumalginat-Perlen (IGB-21) behandelt. Die Perlen von 1–2 mm Durchmesser

enthalten 20 mg $Na^+$, 7,8 mg $K^+$ und 160 mg $Ca^{2+}$ per Gramm Trockensubstanz. Bei einer durchschnittlichen Dosierung von 5,4 g Ca-Alginat je Tag liessen sich die Serum-Phosphatwerte über 6 Monate von 8,5 $\pm$ 1,7 mg% auf 6,1 $\pm$ 1,0 mg%, also um 28%, absenken und befanden sich damit in einem akzeptablen Bereich. Währen die $K^+$-Werte sich dabei nicht signifikant veränderten, wurde eine geringe Verminderung der $Ca^{2+}$-Werte (10%) gemessen (vgl. Tabelle I). Nebenreaktionen wurden nicht beobachtet. Als weiterer Vorteil erwies sich die eindeutige Verminderung der bei der $Al(OH)_3$-Therapie üblichen Verstopfungen.

Analoge Untersuchungen wurden an 5 Patienten mit einem Eisen-dotierten Ca-Alginat (IGB-27) durchgeführt (Fe-Gehalt 40 mg, Ca-Gehalt 140 mg pro Gramm). Es wurden ähnliche Ergebnisse wie die in Tabelle II aufgeführten erhalten.

Tabelle II

Mittelwerte der Serumkonzentrationen von Phosphat, Calcium und Kalium von 10 Patienten mit chronischem Nierenversagen nach Behandlung mit durchschnittlich 5,4 g Ca-Alginat (IGB-21) pro Tag

| | Start | 1 | 2 | 3 | 4 | 5 | 6 Monate |
|---|---|---|---|---|---|---|---|
| Serum-Phosphat mg% | 8,5 ±1,7 | 6,0 ±2,2 | 5,5 ±1,1 | 5,6 ±1,5 | 5,8 ±1,2 | 6,0 ±1,3 | 6,1 ±1,0 |
| Serum-Calcium mmol/l | 2,35 ±0,23 | 2,44 ±0,21 | 2,41 ±0,16 | 2,46 ±0,14 | 2,35 ±0,10 | 2,29 ±0,16 | 2,15 ±0,13 |
| Dosierung des Phosphatbinders g/Tag | | 4,6 ±1,8 | 5,0 ±1,9 | 5,5 ±1,9 | 5,6 ±1,8 | 6,0 ±1,7 | 5,4 ±1,8 |
| N | 10 | 10 | 10 | 10 | 7 | 6 | 5 |

Beispiel 11

Oxalat-Bindungskapazität von Calciumalginat (IGB-21) in Gegenwart von Phosphat in pH- und Zeitabhängigkeit

Zur Ermittlung der Oxalat-Bindungskapazität wurden zwei Reihen von Proben hergestellt:

a) 500 mg Oxalsäure in 190 ml 0,1M Tris-HCl
b) 500 mg Oxalsäure und 960 mg $K_2HPO_4$ in 190 ml 0,1 M Tris-HCl.

Zur Simulierung der pH-Verhältnisse in den einzelnen Darmabschnitten wurden innerhalb jeder Reihe Proben mit folgenden pH-Werten hergestellt: pH 6,0; 65; 7,0; 7,5; 8,0.

Nach genauer Einstellung des pH mit NaOH bzw. HCl wurde mit Puffer auf 200 ml aufgefüllt und jeder Probe 1 g Calciumalginat (IGB-21) zugesetzt. Die Ansätze wurden unter leichter Bewegung bei 37 °C im Wasserbad inkubiert, nach 10, 30, 60 und 120 Minuten jeweils 12 ml Aliquots entnommen, gebildetes Ca-Oxalat abzentrifugiert und vom Überstand jeweisl 10 ml mit $KMnO_4$-Lösung (s. Bestimmungsmethoden) auf freies Oxalat titriert.

Die Ergebnisse zeigt Fig. 7. Zum Vergleich sind jeweils die ohne Phosphat bei pH 7,0 entfernten Oxalatmengen mit aufgeführt. Generell ist die Oxalat-Bindungskapazität von Ca-Alginat (IGB-21) bei Inkubationszeiten ab 30 Minuten bei pH 6,0 am grössten, um dann bis pH 7,5 abzunehmen. Bei pH 7 bindet 1 g Ca-Alginat ca. 165 mg Oxalat. In Gegenwart von Phosphat vermindert sich die Oxalat-Bindungskapazität von IGB-21 um ca. 20%, wenn Reaktionszeiten von 30 Minuten oder mehr zugrundegelegt werden.

Figur 1:

Kinetik der $HPO_4^{2-}$-Bindung durch Calciumalginatgel (8%)

Calciumalginatgel (8%, 5mal gewaschen), das nur an Carbonylgruppen gebundene Calciumionen enthält, wurde im Überschuss mit 5,5 mM $HPO_4^{2-}$ in Tris/HCl-Puffer (pH 8, 0,2 M) bei 37 °C auf dem Rollermixer inkubiert. Von den nach verschiedenen Zeiten gezogenen Proben wurden nach Zentrigugation die $HPO_4^{2-}$-Konzentrationen der Überstände bestimmt. (Vgl. dazu auch Fig. 3)

Figur 2:

Maximale Konzentration von nicht gebundenem $HPO_4^{2-}$ in Abhängigkeit vom pH-Wert

Calciumalginat (8%) wurde im Überschuss jeweils 30 min mit 5,5 mM $HPO_4^{2-}$ in Tris/HCl-Puffer (0,2 M) bei 37 °C im Rollermixer bei verschiedenen pH-Werten inkubiert. Nach Zentrifugation wurden die $HPO_4^{2-}$-Konzentrationen der Überstände bestimmt.

Figur 3:

Kinetik der $HPO_4^{2-}$-Bindung durch Calciumalginat (20%) und Calciumpektat (15%)

Verschiedene Mengen an Calciumalginatgel (20%) und Calciumpektatgel (15%), jeweils dotiert mit einer 1 M $CaCl_2$-Lösung (pH 7,4), wurden mit 10 ml 5,5 mM $HPO_4^{2-}$ in Tris/HCl-Puffer (pH 8, 0,2 M) bei 37 °C im Rollermixer inkubiert. Von den nach verschiedenen Zeiten gezogenen Proben wurde die $HPO_4^{2-}$-Konzentration bestimmt.

Figur 4:
In-vitro-Phosphatbindungskapazität verschiedner Substanzgruppen auf $Al(OH)_3$-Basis (B, C, D), Ca-Alginat-Basis (E, F) und von $Fe(OH)_3$ (G) (vgl. Beispiel 7). Die Säule A repräsentiert die Gesamtmenge an vorgelegtem Phosphat (25 mM).

Figur 5:
Phosphatbindungskapazität verschiedener Phosphatbinder in vitro (per Gramm Substanz) in Abhängigkeit von der Inkubationszeit unter verschiedenen pH-Bedingungen (vgl. Beispiel 8). Es wurden jeweils 0,4 g Phosphat vorgelegt.

Figur 6:
Phosphatelimination durch 2,4 g Ca-Alginat (IGB-21) aus menschlichem Duodenalsaft, der mit 2 g $Na_3PO_4$ angereichert worden war (vgl. Beispiel 9). Der Verlauf der freien Ca-Konzentration über die Versuchsdauer von 120 min ist ebenfalls aufgetragen.

Figur 7:
Oxalatbindungskapazität von Calcium-Alginat (IGB-21) bei verschiedenen pH-Werten in Abhängigkeit von der Inkubationszeit in Gegenwart von Phosphat (offene Säulen) bzw. in Abwesenheit von Phosphat (gepunktete Säulen). Vgl. Beispiel 11.

**Patentansprüche für die Vertragsstaaten: BE · CH/LI · DE · FR · GB · IT · LU · NL · SE**

1. Oral verabreichbares Arzneimittel zur Regulierung des Phosphat- und/oder Calciumspiegels bei chronischer Urämie und/oder zur Regulierung des Oxalat- und/oder Phosphatspiegels bei der Nierensteinprophylaxe, dadurch gekennzeichnet, dass es mindestens ein Calciumsalz oder ein Calciummischsalz einer natürlichen oder chemisch modifizierten polymeren, anionischen Carbonsäure und/oder eines ihrer Schwefelsäureester, wobei das Calciumsalz das 0,5- bis 5fache der stöchiometrisch erforderlichen Menge an Calciumionen enthält, die Kationen in dem Calciummischsalz aus der Gruppe Calcium, Eisen und/oder Kationen von Spurenelementen ausgewählt werden, die Summe der Kationen im Calciummischsalz im Bereich des 0,5- bis 5fachen der stöchiometrisch erforderlichen Menge liegt, es die 0,45- bis 4,95fache Menge der stöchiometrisch erforderlichen Menge an Calciumionen und das 0,05- bis 2,5fache der stöchiometrisch erforderlichen Menge an Eisenionen und/oder Kationen von Spurenelementen enthält, und übliche Zusatzstoffe und/oder Trägerstoffe enthält und dass es in Form von Perlen, Tabletten, Kapseln, Pulver, Dragees oder Pillen vorliegt.

2. Arzneimittel nach Anspruch 1, dadurch gekennzeichnet, dass es ein Calciumsalz oder Calciummischsalz einer Polymannuronsäure, Polygalacturonsäure, Polyglucuronsäure, Polyguluronsäure, eines Oxidationsprodukts von Homoglykanen, eines Oxidationsprodukts von Heteroglykanen oder dren Gemische enthält.

3. Arzneimittel nach Anspruch 1, dadurch gekennzeichnet, dass es als Calciumsalz das stöchiometrische Calciumsalz enthält oder dass das Calcium in einer Menge im Bereich des 0,5- bis 2fachen, vorzugsweise 0,8- bis 2fachen, der stöchiometrisch erforderlichen Menge vorhanden ist.

4. Arzneimittel nach Anspruch 1, 2 oder 3, dadurch gekennzeichnet, dass es Calciumalginat und/oder Calciumpektat enthält.

5. Arzneimittel nach Anspruch 1 oder 2, dadurch gekennzeichnet, dass es ein Calciummischsalz enthält, in dem das Calcium in einer Menge des 0,2- bis 1,5fachen der stöchiometrisch erforderlichen Menge durch Eisenionen und/oder Kationen von Spurenelementen ersetzt ist.

6. Verwendung von mindestens einem Calciumsalz oder Calciummischsalz einer natürlichen oder chemisch modifizierten polymeren, anionischen Carbonsäure und/oder eines ihrer Schwefelsäureester, wobei das Calciumsalz das 0,5- bis 5fache der stöchiometrisch erforderlichen Menge an Calciumionen enthält, die Kationen in dem Calciummischsalz aus der Gruppe Calcium, Eisen und/oder Kationen von Spurenelementen ausgewählt werden, die Summe der Kationen im Calciummischsalz im Bereich des 0,5- bis 5fachen der stöchiometrisch erforderlichen Menge liegt, es die 0,45- bis 4,95fache Menge der stöchiometrisch erforderlichen Menge an Calciumionen und das 0,05- bis 2,5fache der stöchiometrisch erforderlichen Menge an Eisenionen und/oder Kationen von Spurenelementen enthält, und üblichen Zusatzstoffen und/oder Trägerstoffen zur Herstellung eines oral verabreichbaren Arzneimittels zur Regulierung des Phosphat- und/oder Calciumspiegels bei chronischer Urämie und/oder zur Regulierung des Oxalat- und/oder Phosphatspiegels bei der Nierensteinprophylaxe.

7. Verwendung nach Anspruch 6, dadurch gekennzeichnet, dass ein Calciumsalz oder Calciummischsalz einer Polymannuronsäure, Polygalacturonsäure, Polyglucuronsäure, Polyguluronsäure, eines Oxidationsprodukts von Homoglykanen, eines Oxidationsprodukts von Heteroglykanen oder deren Gemische verwendet werden.

8. Verwendung nach Anspruch 6, dadurch gekennzeichnet, dass als Calciumsalz das stöchiometrische Calciumsalz oder ein Calciumsalz, in dem das Calcium in einer Menge im Bereich der 0,5- bis 2fachen, vorzugsweise 0,8- bis 2fachen, stöchiometrisch erforderlichen Menge vorhanden ist, verwendet wird.

9. Verwendung nach Anspruch 6, 7 oder 8, dadurch gekennzeichnet, dass Calciumalginat und/oder Calciumpektat verwendet werden.

10. Verwendung nach Anspruch 6 oder 7, dadurch gekennzeichnet, dass ein Calciummischsalz, in dem das Calcium in einer Menge des 0,2- bis 1,5fachen der stöchiometrisch erforderlichen Menge durch Eisenionen und/oder Kationen von Spurenelementen ersetzt ist, verwendet wird.

11. Verwendung nach einem der vorhergehenden Ansprüche, dadurch gekennzeichnet, dass es in Form von Perlen, Tabletten, Kapseln, Pulver, Dragees oder Pillen verwendet wird.

12. Calciummischsalz, dadurch gekennzeichnet, dass es ein Calcium-Eisen-Alginat und/oder -Pektat enthält.

**Ansprüche für den Vertragsstaat: AT**

1. Verfahren zur Herstellung eines in einem oral verabreichbaren Arzneimittel zur Regulierung des Phosphat- und/oder Calciumspiegels bei chronischer Urämie und/oder zur Regulierung des Oxalat- und/oder Phosphatspiegels bei der Nierensteinprophylaxe verwendbaren Calciummischsalzes einer natürlichen oder chemisch modifizierten polymeren, anionischen Carbonsäure und/oder eines ihrer Schwefelsäureester, wobei die Kationen in dem Calciummischsalz aus der Gruppe Calcium, Eisen und/oder Kationen von Spurenelementen ausgewählt sind und wobei die Summe der Kationen im Calciummischsalz im Bereich des 0,5- bis 5fachen der stöchimetrisch erforderlichen Menge liegt und es die 0,45- bis 4,95fache Menge der stöchimetrisch erforderlichen Menge an Calciumionen und das 0,05- bis 2,5fache der stöchimetrisch erforderlichen Menge an Eisenionen und/oder Kationen von Spurenelementen enthält, dadurch gekennzeichnet, dass man zu einer 2- bis 20%igen, wässerigen Lösung einer natürlichen oder chemisch modifizierten polymeren, anionischen Carbonsäure und/oder eines ihrer Schwefelsäureester, ein Alkalihydroxid bis zu einem pH-Wert von 5,0 bis 8,0 hinzugibt, die Lösung gegebenenfalls 1 bis 2 Stunden bei Raumtemperatur rührt, die so erhaltene Lösung bei einer Temperatur im Bereich von 0 bis 50 °C in eine 0,2 bis 2M Lösung eines physiologisch annehmbaren Calciumsalzes, deren pH-Wert mit Alkalihydroxid auf einen Wert im Bereich von 2 bis 8 eingestellt wurde, oder eine 0,01 bis 1M wässerige Lösung eines physiologisch annehmbaren Eisen-II-Salzes und/oder gegebenenfalls eine 0,01 bis 0,5M wässerige Lösung physiologisch annehmbarer Salze von Spurenelementen eintropft, wobei dieser Lösung Calciumionen in einer Konzentration von 0,01 bis 2M zugesetzt werden können, das Reaktionsgemisch anschliessend 1 bis 20 Stunden bei einer Temperatur von 2 bis 50 °C stehen lässt, das Reaktionsprodukt abfiltriert, den erhaltenen Niederschlag bis zum erforderlichen Restgehalt an freine (Spuren-)Ionen wäscht und anschliessend trocknet.

2. Verfahren nach Anspruch 1, dadurch gekennzeichnet, dass man eine Polycarbonatsäure verwendet, die ausgewählt ist aus der Gruppe Polymannuronsäure, Polygalacturonsäure, Polyglucuronsäure, Polyguluronsäure, den Oxidationsprodukten von Homoglykanen, den Oxidationsprodukten von Heteroglykanen oder ihrer Schwefelsäureester oder deren Gemisch.

3. Verfahren nach Anspruch 2, dadurch gekennzeichnet, dass man das Mischsalz Calcium-Eisen-Alginat herstellt, indem man als Polycarbonsäure Alginsäure verwendet.

4. Verfahren nach Anspruch 2, dadurch gekennzeichnet, dass man Calcium-Eisen-Pektat herstellt, indem man als Polycarbonsäure Pektinsäure verwendet.

5. Verfahren nach einem der Ansprüche 1 bis 4, dadurch gekennzeichnet, dass man als physiologisch annehmbare Salzlösungen des Calciums, des Eisens oder der Spurenelemente anorganische Salze, wie die Chloride oder die Carbonate, oder organische Salze, wie die Acetate, Ascorbate, Citrate, Gluconate, Lactate, Levulinate, Malonate, Pantothenate, Saccharate oder Tartrate verwendet.

**Claims for the contracting states: BE · CH/LI · DE · FR · GB · IT · LU · NL · SE**

1. Orally administered medicament for regulating the phosphate and/or calcium level in chronic uraemia and/or for regulating the oxalate and/or phosphate level in kidney stone prophylaxis, characterised in that it contains at least one calcium salt or mixed calcium salt of a natural or chemically modified polymeric anionic carboxylic acid and/or of one its sulphuric acid esters, the calcium salt containing from 0.5 to 5 times the stoichiometrically required quantity of calcium ions, the cations in the mixed calcium salt being selected from calcium, iron and/or cations of trace elements, the sum of catoins in the mixed calcium salths being in the range of from 0.5 to 5 times the stoichiometrically required quantity and the mixed salt containing from 0.45 to 4.95 times the stoichiometrically required quantity of calcium ions and from 0.05 to 2.5 times the stoichiometrically required quantity of iron ions and/or cations of trace elements, and the medicament containing the usual additives and/or carriers and in that it is present in the form of pellets, tablets, capsules, powders, dragees or pills.

2. Medicament according to claim 1, characterised in that it contains a calcium salt or mixed calcium salt of a polymannuronic acid, a polygalacturonic acid, a polyglucuronic acid or a polyguluronic acid or of an oxidation product of homoglycans or of an oxidation product of heteroglycans or mixtures thereof.

3. Medicament according to claim 1, characterised in that the calcium salt contained in it is the stoichiometric calcium slt or that the calcium is present in a quantity in the range of from 0.5 to 2 times, preferably from 0.8 to 2 times the stoichiometrically required quantity.

4. Medicament according to claim 1, 2 or 3, characterised in that it contains calcium alginate and/or calcium pectate.

5. Medicament according to claim 1 or 2, characterised in that it contains a mixed calcium salt in which from 0.2 to 1.5 times the stoichiometrically required quantity of calcium is replaced by ions of iron and/or cations of trace elements.

6. Use of at least one calcium salt or mixed calcium salt of a natural or chemically modified polymeric anionic carboxylic acid and/or of one of its sulphuric acid esters, the calcium salt containing from 0.5 to 5 times the stoichiometrically required quantity of calcium ions, the cations in the

mixed calcium salt being selected from calcium iron and/or cations of trace elements, the sum of cations in the mixed calcium slat being in the range of from 0.5 to 5 times the stoichiometrically required quantity and the mixed salt containing from 0.45 to 4.95 times the stoichiometrically required quantity of calcium ions and from 0.05 to 2.5 times the stoichiometrically required quantity of iron ions and/or cations of trace elements, and the usual additives and/or carriers being present for the preparatoin sof a medicament which can be administered orally for regulating the phosphate and/or calcium level in chronic uraemia and/or for regulating the oxalate and/or phosphate level in kidney stone prophylaxis.

7: use according to claim 6, characterised in that a calcium salt or mixed calcium salt of a polymannuronic acid, a polygalacturonic acid, a polyglucuronic acid or a polyguluronic acid or of an oxidation product of homoglycans or of an oxidation product of heteroglycans or mixtures thereof are used.

8. Use according to claim 6, characterised in that the calcium salt used is the stoichiometric calcium salt or a calcium salt in which the calcium is present in a quantity of from 0.5 to 2 times, preferably from 0.8 to 2 times the stoichiometrically required quantity.

9. Use according to claim 6, 7 or 8, characterised in that calcium alginate and/or calcium pectate is used.

10. Use according to claim 6 or 7, characterised in that a mixed calcium salt is used in which from 0.2 to 1.5 times the stoichiometrically required quantity of calcium is replaced by iron ions and/or cations of trace elements.

11. Use according to one of the preceding claims, characterised in that it is used in the form of pellets, tablets, capsules, powders, dragees or pills.

12. Mixed calcium salt, characterised in that it contains a calcium-iron alginate and/or calcium-iron pectate.

**Claims for the contracting State: AT**

1. Process for the preparation of a mixed calcium salt of a natural or chemically modified polymeric anionic carboxylic acid and/or one of its sulphuric acid esters which can be used in an orally administered medicament for regulating the phospahte and/or calcium level in chronic uraemia and/or for regulating the oxalate and/or phosphate level in kidney stone prophylaxis, the cations in the mixed calcium salt being selected from calcium, iron and/or cations of trace elements and the sum fo cations in the mixed calcium salt being in the range of from 0.5 to 5 times the stoichiometrically required quantity and the salt containing from 0.45 to 4.95 times the stoichiometrically required quantity of calcium ions and from 0.05 to 2.5 times the stoichiometrically required quantity of iron ions and/or cations of trace elements, characterised in that an alkali metal hydroxide is added to a 2 to 20% aqueous solution of a natural or chemically modified polymeric anionic carboxylic acid and/or one of its sulphiric acid esters until the pH has a value from 5.0 to 8.0, the solution is optionally stirred for 1 to 2 hours at room temperature, the resulting solution is introduced dropwise at a temperature from 0 °C to 50 °C into a 0.2 to 2M solution of a physiologically acceptable calcium salt whose pH has been adjusted to a value from 2 to 8 with alkali metal hydroxide or into a 0.01 to 1M aqueous solution of a physiologically acceptable iron-II salt and/or optionally a 0.01 to 0.5M aqueous solution of physiologically acceptable salts of trace elements, to which solution there may be added calcium ions at a concentration of from 0.01 to 2M, and the reaction mixture is then left to stand for 1 to 20 hours at a temperature from 2 °C to 50 °C, the reaction product is filtered off, and the precipitate obtained is washed until the quantity of free (trace) ions has been reduced to the required residual level and is then dried.

2. Process according to claim 1, characterised in that the polycarboxylic acid used is selected from polymannuronic acid, polygalacturonic acid, polyglucuronic acid, polyguluronic acid, the oxidation products of homoglycans, the oxidation products of heteroglycans or the sulphuric acid esters of these compounds or mixutres thereof.

3. Process according to claim 2, characterised in that the mixed calcium-iron alginate salt is prepared from alginic acid as the polycarboxylic acid.

4. Process according to claim 2, characterised in that calcium-iron pectate is prepared by using pectic acid as the polycarboxylic acid.

5. Process according to one of the claims 1 to 4, characterised in that the physiologically acceptable salt solutions of calcium, of iron or of trace elements used are solutions of inorganic salts such as chlorides or carbonates or organic salts such as acetates, ascorbates, citrates, gluconates, lactates, levulinates, malonates, pantothenates, saccharates or tartrates.

**Revendications pour les états contractants: BE · CH/LI · DE · FR · GB · IT · LU · NL · SE**

1. Médicament administrable par voie orale pour la régulation du taux de phosphate et/ou du taux de calcium dans l'urémie chronique et/ou pour la régulation du taux d'oxalate et/ou du taux de phosphate dans la prophylaxie des calculs rénaux, caractérisé en ce qu'il contient au moins un sel de calcium ou un sel mixte de calcium d'un acide carboxylique anionique polymérique naturel ou chimiquement modifié et/ou l'un de ses esters d'acide sulfurique, le sel de calcium contenant 0,5 à 5 fois la quantité stoechiométriquement nécessaire des ions de calcium, les cations du sel mixte de calcium étant choisis dans le groupe du calcium, du fer et/ou des cations d'oligo-éléments, la somme des cations du sel mixte de calcium se situant dans l'intervalle de 0,5 à 5 fois la quantité stoechiométriquement nécessaire, ledit sel mixte

contenant 0,45 à 4,95 fois la quantité stoechiométriquement nécessaire d'ions calcium et 0,05 à 2,5 fois la quantité stoechiométriquement nécessaire d'ions fer et/ou de cations d'oligo-éléments, et renfermant des additifs et/ou véhicules courants, et en ce qu'il se présente sous forme de perles, comprimés, capsules, poudres, dragées ou pilules.

2. Médicament selon la revendication 1, caractérisé en ce qu'il contient un sel de calcium ou un sel mixte de calcium d'un acide polymannuronique, d'un acide polygalacturonique, d'un acide polyglucuronique, d'un acide polyguluronique, d'un produit d'oxydation d'homoglucanes, d'un produit d'oxydation d'hétéroglucanes ou de leurs mélanges.

3. Médicament selon la revendication 1, caractérisé en ce qu'il contient en tant que sel de calcium la quantité stoechiométrique de sel de calcium ou que le calcium est présent en une quantité dans l'intervalle de 0,5 à 2 fois, de préférence 0,8 à 2 fois la quantité stoechiométriquement nécessaire.

4. Médicament selon la revendication 1, 2 ou 3, caractérisé en ce qu'il contient un alginate de calcium et/ou un pectate de calcium.

5. Médicament selon la revendication 1 ou 2, caractérisé en ce qu'il contient un sel mixte de calcium dans lequel le calcium est remplacé en une quantité de 0,2 à 1,5 fois la quantité stoechiométriquement nécessaire par des ions fer et/ou des cations d'oligo-éléments.

6. Utilisation d'au moins un sel de calcium ou sel mixte de calcium d'un acide carboxylique anionique polymérique naturel ou chimiquement modifié et/ou l'un de ses esters d'acide sulfurique, le sel de calcium contenant 0,5 à 5 fois la quantité stoechiométriquement nécessaire des ions de calcium, les cations du sel mixte de calcium étant choisis dans le groupe du calcium, du fer et/ou des cations d'oligo-éléments, la somme des cations du sel mixte de calcium se situant dans l'intervalle de 0,5 à 5 fois la quantité stoechiométriquement nécessaire, ledit sel mixte de calcium contenant 0,45 à 4,95 fois la quantité stoechiométriquement nécessaire d'ions calcium et 0,05 à 2,5 fois la quantité stoechiométriquement nécessaire d'ions fer et/ou de cations d'oligo-éléments, et renfermant des additifs et/ou véhicules courants, pour la préparation d'un médicament administrable par voie orale pour la régulation du taux de phosphate et/ou du taux de calcium dans l'urémie chronique et/ou pour la régulation du taux d'oxalate et/ou du taux de phosphate dans la prophylaxie des calculs rénaux.

7. Utilisation selon la revendication 6, caractérisée en ce qu'on utilise un sel de calcium ou sel mixte de calcium d'un acide polymannuronique, d'un acide polygalacturonique, d'un acide polyglucuronique, d'un acide polyguluronique, d'un produit d'oxydation d'homoglucanes, d'un produit d'oxydation d'hétéroglucanes ou de leurs mélanges.

8. Utilisation selon la revendication 6, caractérisée en ce qu'on utilise en tant que sel de calcium la quantité de sel de calcium stoechiométrique ou un sel de calcium dans lequel le calcium est présent en une quantité dans l'intervalle de 0,5 à 2 fois, de préférence de 0,8 à 2 fois la quantité stoechiométriquement nécessaire.

9. Utilisation selon la revendication 6, 7 ou 8, caractérisée en ce qu'on utilise un alginate de calcium et/ou un pectate de calcium.

10. Utilisation selon la revendicatoin 6 ou 7, caractérisée en ce qu'on utilise un sel mixte de calcium dans lequel le calcium est remplacé en une quantité de 0,2 à 1,5 fois la quantité stoechiométriquement nécessaire par des ions fer et/ou des cations d'oligo-éléments.

11. Utilisation selon l'une des revendications précédentes, caractérisée en ce que le médicament préparé se présente sous forme de perles, comprimés, capsules, poudres, dragées ou pilules.

12. Sel mixte de calcium, caractérisé en ce qu'il contient un alginate de calcium-fer et/ou un pectate de calcium-fer.

**Revendications pour l'état contractant: AT**

1. Procédé pour la préparation d'un sel mixte de calcium d'un acide carboxylique anionique polymérique naturel ou chimiquement modifié et/ou l'un de ses esters d'acide sulfurique, utilisable dans un médicament administrable par voie orale pour la régulation du taux de phosphate et/ou du taux de calcium dans l'urémie chronique et/ou pour la régulation du taux d'oxalate et/ou du taux de phosphate dans la prophylaxie des calculs rénaux, les cations du sel mixte de calcium étant choisis dans le groupe du calcium, du fer et/ou des cations d'oligo-éléments, la somme des cations du sel mixte de calcium se situant dans l'intervalle de 0,5 à 5 fis la quantité stoechiométriquement nécessaire et ledit sel mixte de calcium contenant 0,45 à 4,95 fois la quantité stoechiométriquement nécessaire d'ions de calcium et 0,05 à 2,5 fois la quantité stoechiométriquement nécessaire d'ions fer et/ou de cations d'oligo-éléments, caractérisé en ce qu'à une solution aqueuse à 2–20% d'un acide carboxylique anionique polymérique naturel ou chimiquement modifié ou l'un de ses esters d'acide sulfurique, on ajoute un hydroxyde alcalin jusqu'à une valeur de pH de 5,0 à 8,0, on agite le cas échéant la solution pendant 1 à 2 heures à la température ambiante, on introduit goutte à goutte la solution ainsi obtenue, à une température dans l'intervalle de 0 à 50°C dans une solution 0,2 à 2M d'un sel de calcium physiologiquement acceptable, dont le pH a été établi avec un hydroxyde alcalin à une valeur dans l'intervalle de 2 à 8, ou une solution aqueuse 0,01 à 1M d'un sel ferreux physiologiquement acceptable et/ou le cas échéant une solution aqueuse 0,01 à 0,5M de sels d'oligo-éléments physiologiquement acceptables, à cette solution pouvant être ajoutés des ions calcium en une concentration de 0,01 à 2M, on laisse consécutivement reposer le mélange réactionnel pendant

1 à 20 heures à une température de 2 à 50 °C, on sépare le produit réactionnel par filtration, on lave le précipité obtenu jusqu'à la teneur résiduaire en ions libres (à l'état de traces) nécessaire et, consécutivement, on le sèche.

2. Procédé selon la revendication 1, caractérisé en ce qu'on utilise un acide polycarboxylique qui est choisi dans le groupe acide polymannuronique, acide polygalacturonique, acide polyglucuronique, acide polyguluronique, les produits d'oxydation d'homoglucanes, les produits d'oxydation d'hétéroglucanes ou leurs esters d'acide sulfurique ou un mélange de ceux-ci.

3. Procédé selon la revendication 2, caractérisé en ce qu'on prépare le sel mixte alginate de calcium-fer, en utilisant à cet effet comme acide polycarboxylique, de l'acide alginique.

4. Procédé selon la revendication 2, caractérisé en ce qu'on prépare un pectate de calcium-fer, en utilisant à cet effet comme acide polycarboxylique, de l'acide pectique.

5. Procédé selon l'une des revendications 1 à 4, caractérisé en ce qu'on utilise comme solutions de sels physiologiquement acceptables, des sels inorganiques du calcium, du fer ou des oligo-éléments, tels que les chlorures ou les carbonates, ou des sels organiques, tels que des acétates, ascorbates, citrates, gluconates, lactates, lévulinates, malonates, pantothénates, saccharates ou tartrates.

FIG.1

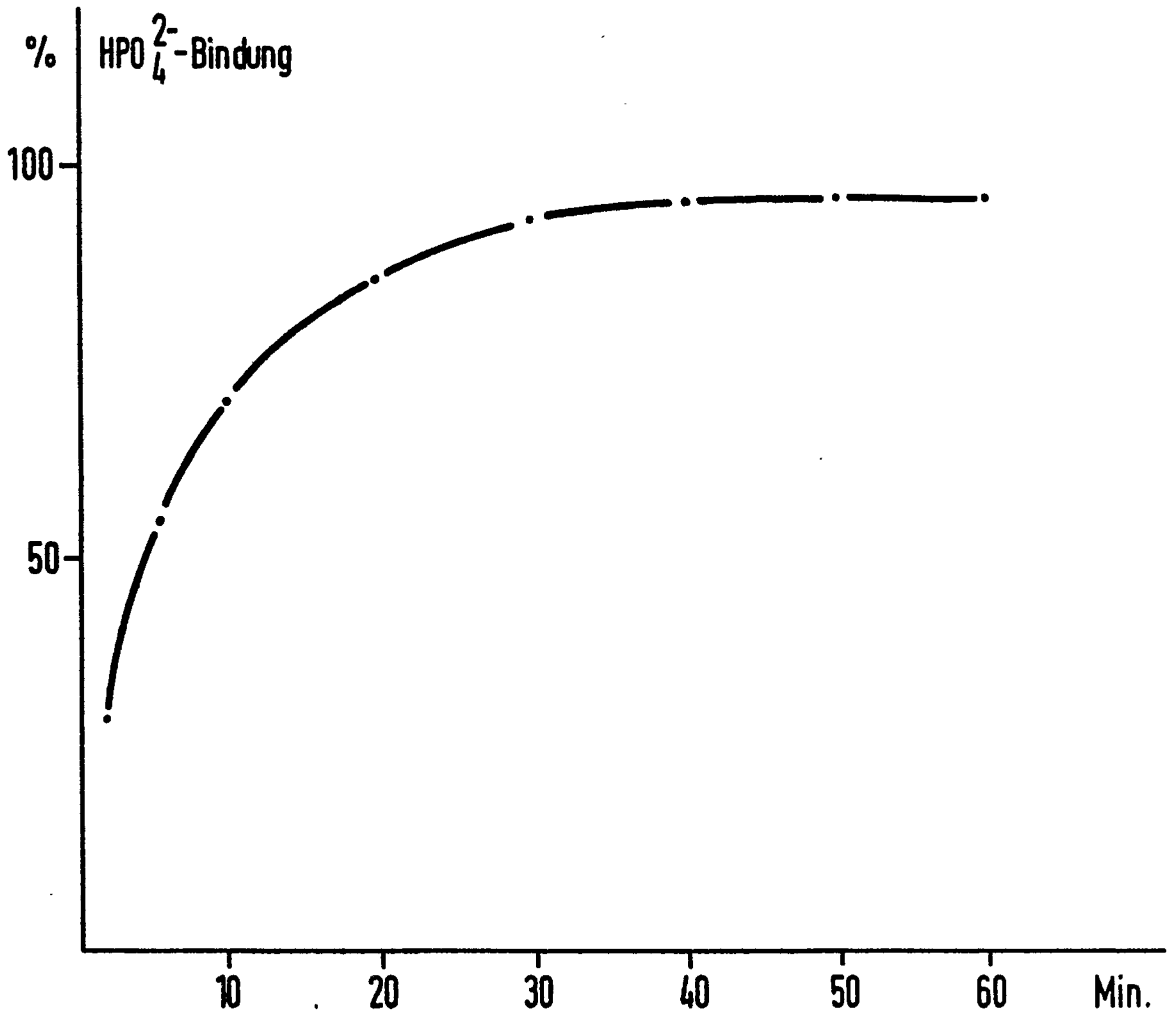

FIG.2

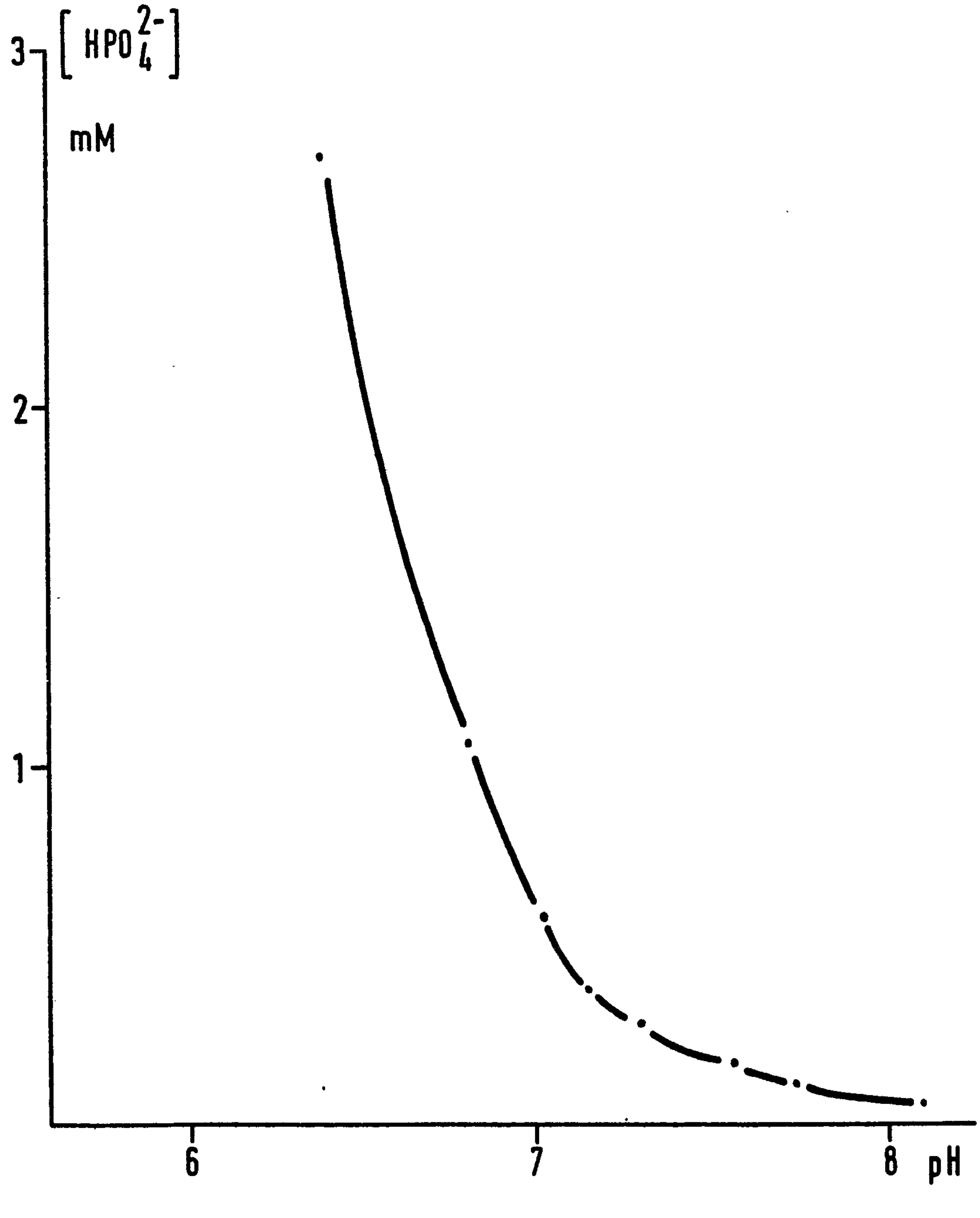

FIG.3

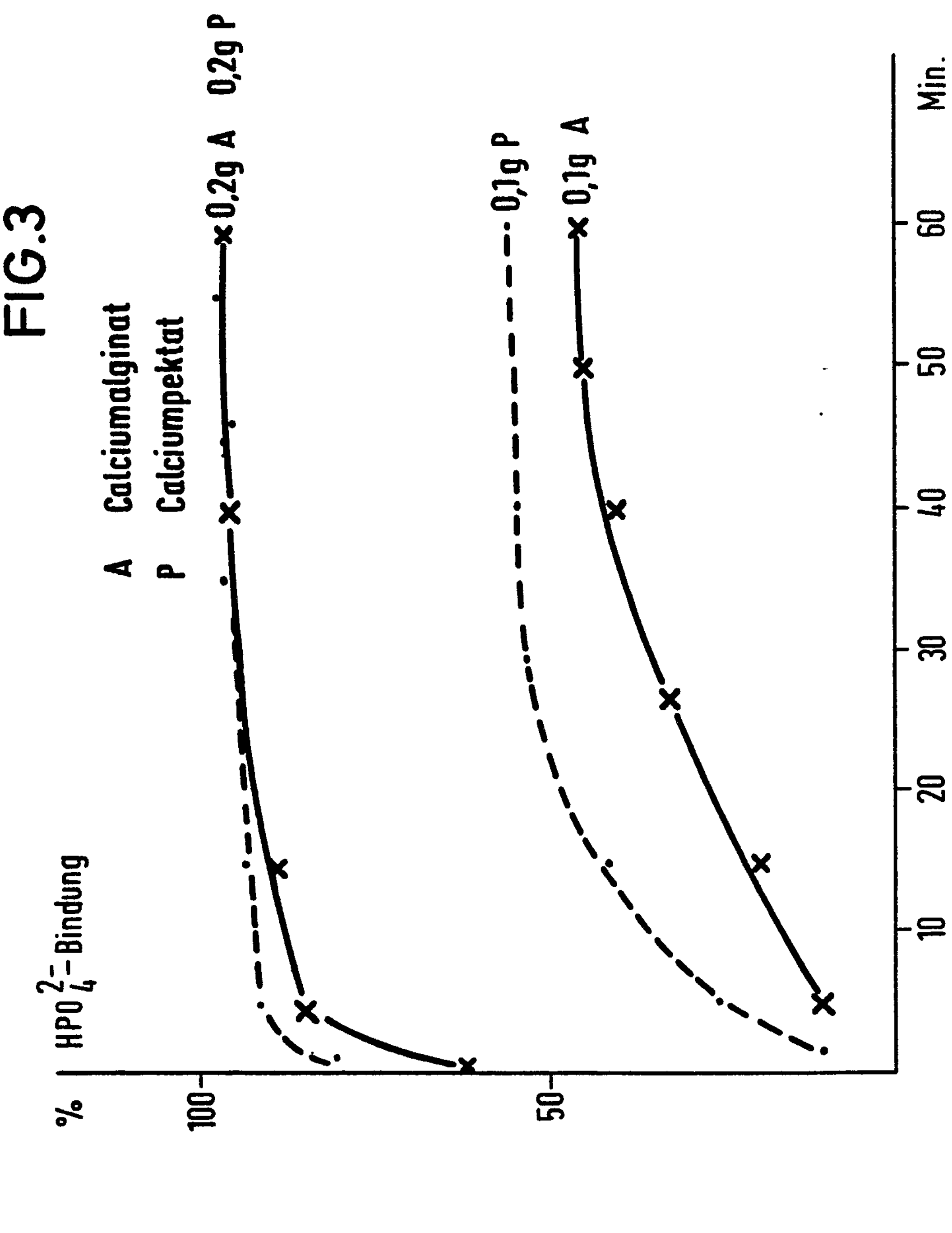

%
100
50
$HPO_4^{2-}$-Bindung
A Calciumalginat
P Calciumpektat
0,2g A
0,2g P
0,1g P
0,1g A
10
20
30
40
50
60
Min.

FIG.4

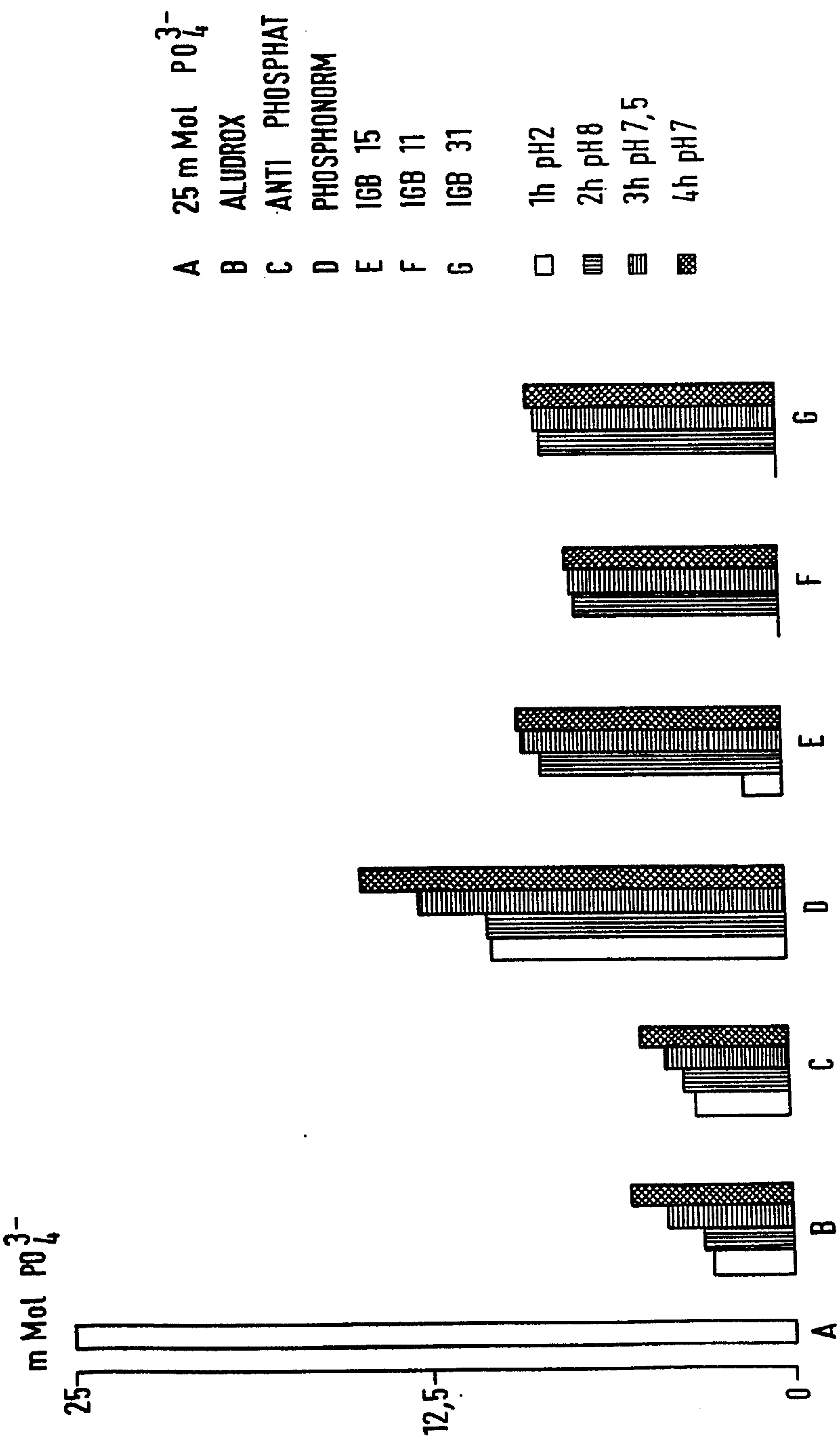
m Mol $PO_4^{3-}$
25
12,5
0
A
B
C
D
E
F
G
A 25 m Mol $PO_4^{3-}$
B ALUDROX
C ANTI PHOSPHAT
D PHOSPHONORM
E IGB 15
F IGB 11
G IGB 31
1h pH2
2h pH8
3h pH7,5
4h pH7

FIG.5

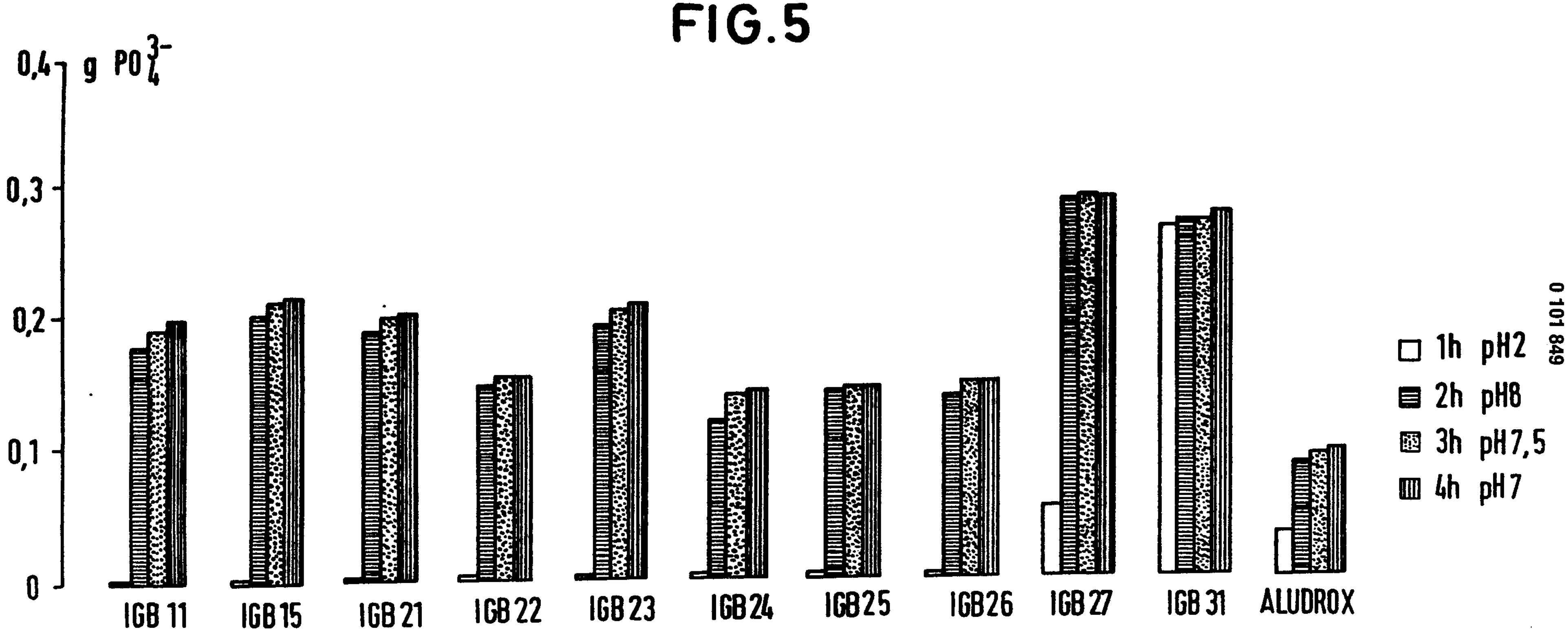
0,4
0,3
0,2
0,1
0
g $PO_4^{3-}$
IGB 11
IGB 15
IGB 21
IGB 22
IGB 23
IGB 24
IGB 25
IGB 26
IGB 27
IGB 31
ALUDROX
1h pH2
2h pH8
3h pH7,5
4h pH7

FIG.6

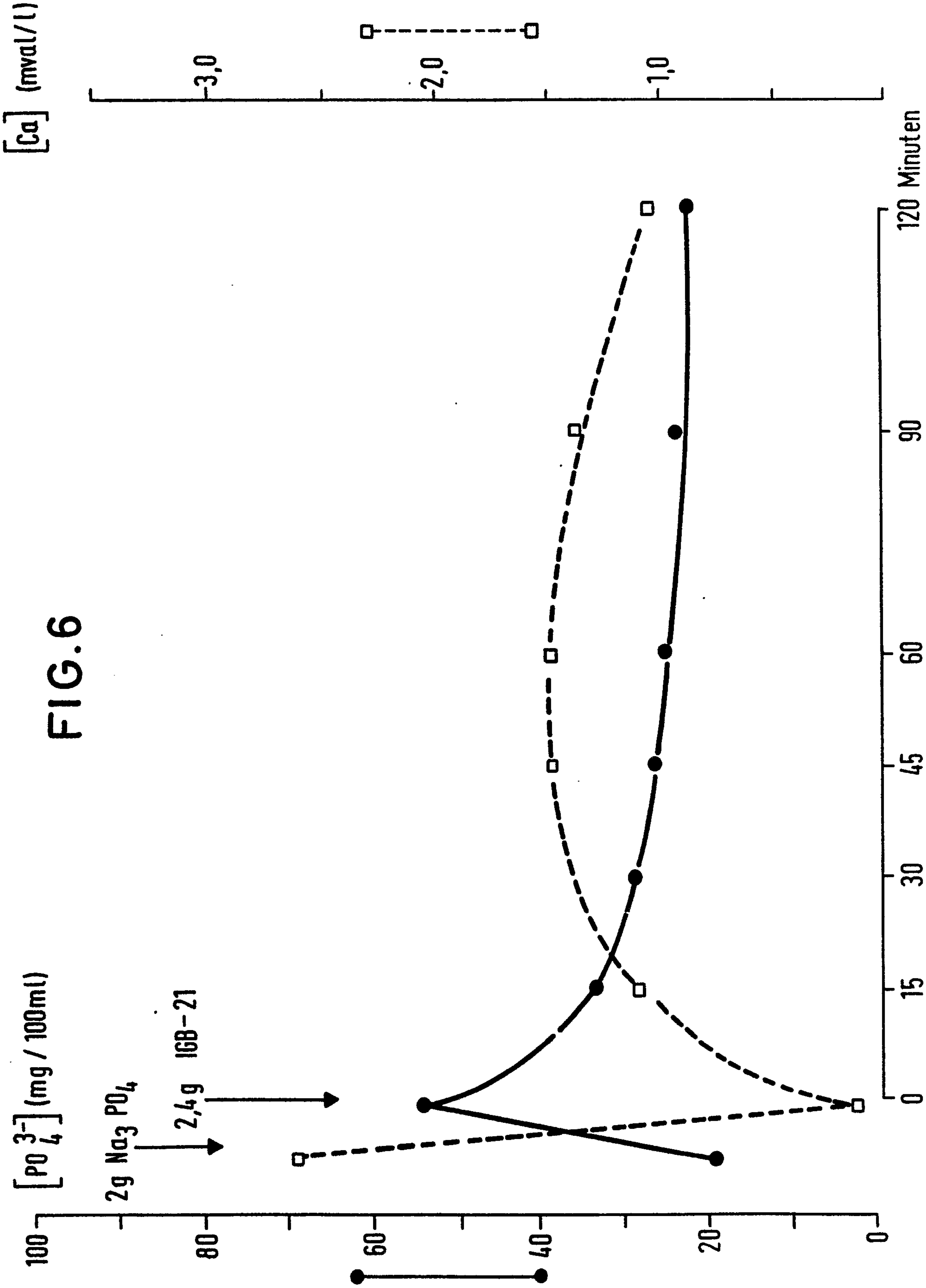

[PO 3- 4] (mg / 100ml)
2g Na3 PO4
2,4g IGB-21
100
80
60
40
20
0
0
15
30
45
60
90
120 Minuten
[Ca] (mval/l)
3,0
2,0
1,0

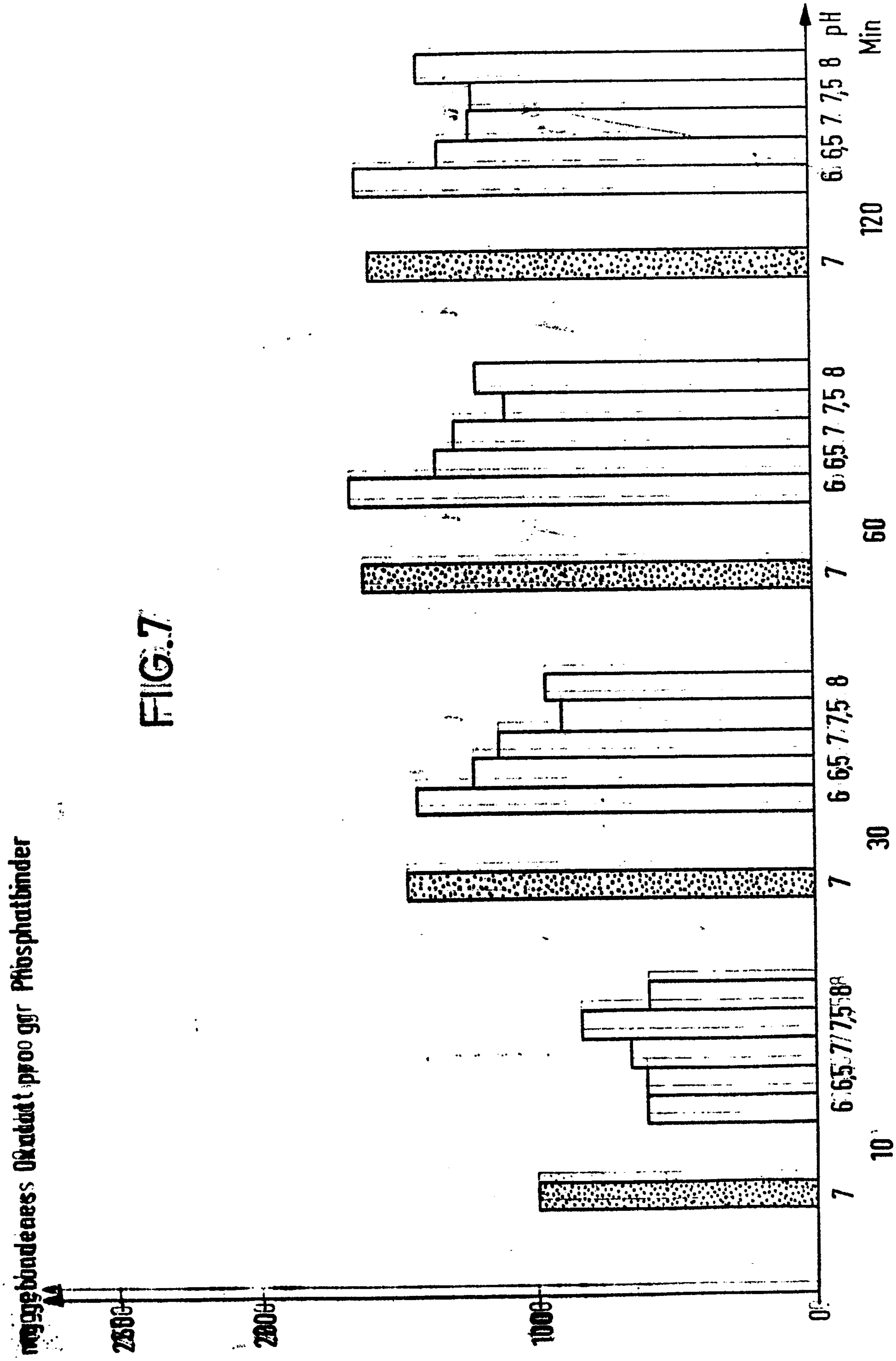
mg gebundenes Oxalat pro gr Phosphatbinder
0
1000
2000
2500
7
6 6,5 7 7,5 8
10
7
6 6,5 7 7,5 8
30
7
6 6,5 7 7,5 8
60
7
6 6,5 7 7,5 8
120
pH
Min

FIG. 7